# EUROPEAN PATENT SPECIFICATION

(11) **EP 2 758 538 B1**
(45) Date of publication and mention of the grant of the patent: **31.10.2018**
(21) Application number: 12834150.0
(22) Date of filing: 20.09.2012
(51) Int. Cl.: C12Q 1/6869, C12Q 1/6886

(54) **METHODS FOR ANALYZING HETEROGENEOUS SAMPLES**
VERFAHREN ZUR ANALYSE HETEROGENER PROBEN
PROCÉDÉS POUR ANALYSER DES ÉCHANTILLONS HÉTÉROGÈNES

(30) Priority: 22.09.2011 US 201161537875 P; 01.11.2011 US 201161554086 P; 08.03.2012 US 201261608442 P
(43) Date of publication of application: 30.07.2014
(73) Proprietor: LINEAGE BIOSCIENCES, INC., Vancouver BC V5Y 0A1 (CA)
(72) Inventor: SELIGSON, Dan, Palo Alto, CA 94301 (US); SNYDER, Thomas, Palo Alto, CA 94306 (US)
(74) Representative: Patent Boutique LLP
(86) International application number: PCT/US2012/056416
(87) International publication number: WO 2013/043922

(56) References cited:
- WO-A1-99/18231
- WO-A1-2011/057061
- WO-A2-02/061148
- WO-A2-2009/025852
- WO-A2-2009/102470
- US-A1- 2004 241 764
- US-A1- 2010 305 000
- US-A1- 2010 330 035
- US-B1- 7 745 132
- CHAO CHENG ET AL: "Quantification of circulating cell-free DNA in the plasma of cancer patients during radiation therapy", CANCER SCIENCE, JAPANESE CANCER ASSOCIATION, TOKYO, JP, vol. 100, no. 2, 16 December 2009 (2009-12-16), pages 303-309, XP008153413, ISSN: 1347-9032, DOI: 10.1111/J.1349-7006.2008.01021.X [retrieved on 2009-12-16]
- ABD EL ET AL: "Clinicopathological Significance and Prognostic Importance of Circulating Plasma DNA Expression in Advanced Non-Small Cell Lung Cancer and its Efficacy as a Diagnostic Tool Significance and Prognostic Importance of Circulating Plasma DNA Expression in Advanced Non-Small Cell Lung Cancer and its Effi", LIFE SCIENCE JOURNAL LIFE SCIENCE JOURNAL, vol. 8, 1 January 2011 (2011-01-01), pages 143-150, XP055176454,
- MARCO AGOSTINI ET AL: "Circulating Cell-Free DNA: A Promising Marker of Pathologic Tumor Response in Rectal Cancer Patients Receiving Preoperative Chemoradiotherapy", ANNALS OF SURGICAL ONCOLOGY, SPRINGER-VERLAG, NE, vol. 18, no. 9, 17 March 2011 (2011-03-17) , pages 2461-2468, XP019945160, ISSN: 1534-4681, DOI: 10.1245/S10434-011-1638-Y
- KOUNALAKIS NICOLE ET AL: "Tumor cell and circulating markers in melanoma: diagnosis, prognosis, and management", CURRENT ONCOLOGY REPORTS, CURRENT SCIENCE, GB, vol. 7, no. 5, 1 September 2005 (2005-09-01), pages 377-382, XP009128179, ISSN: 1523-3790, DOI: 10.1007/S11912-005-0065-2
- HEIDI SCHWARZENBACH ET AL: "Detection and Monitoring of Cell-Free DNA in Blood of Patients with Colorectal Cancer", ANNALS OF THE NEW YORK ACADEMY OF SCIENCES, vol. 1137, no. 1, 1 August 2008 (2008-08-01), pages 190-196, XP055176456, ISSN: 0077-8923, DOI: 10.1196/annals.1448.025
- BOSSOLA ET AL.: 'Circulating Bacterial-Derived DNA Fragments and Markers of Inflammation in Chronic Hemodialysis Patients.' CLIN. J. AM. SOC.NEPHROL. vol. 4, February 2009, pages 379 - 385, XP003027780

## Description

### BACKGROUND OF THE INVENTION

Nucleic acids associated with certain pathological or physiological processes are sometimes released into the blood or other bodily fluids of a subject. For example, nucleic acids derived from tumors may be found in bodily fluids of subjects suffering from cancer. Additionally, nucleic acids derived from an unborn fetus may be found in the bodily fluids of pregnant subjects, while nucleic acids derived from donor organs may be found in certain bodily fluids of transplant recipients. As a result, bodily fluids of a subject may contain a heterogeneous mix of nucleic acids from different genomic sources.

Noise or background signal from the genome of a host subject can often make it difficult to detect or distinguish a foreign genome within a biological sample taken from the host. There is thus a need for improved methods for the detection of certain nucleic acids within a heterogeneous sample.

### SUMMARY OF THE INVENTION

In accordance with one aspect of the present invention, there is provided a method comprising the steps of:
a. detecting a quantity of nucleic acids from cancer tissue in a biological sample obtained from a subject with cancer, the biological sample comprising circulating cell-free nucleic acids, wherein the circulating cell-free nucleic acids comprise nucleic acids from cancer tissue and normal tissue, wherein the circulating cell-free nucleic acids are mRNA or DNA; and wherein the subject has been administered a therapeutic regimen, wherein the detecting comprises conducting a sequencing reaction on the circulating cell-free nucleic acids; and
b. modifying the therapeutic regimen to be administered to the subject based on the quantity of the nucleic acids from the cancer tissue in the biological sample, wherein the therapeutic regimen is increased if the quantity of the nucleic acids from the cancer tissue in the biological sample is greater than 0.5% of the total nucleic acids in the biological sample.

In another aspect, there is provided a method comprising the steps of:
a. detecting a first quantity of cell-free nucleic acids in a biological sample obtained from a subject with cancer at a first point in time, the biological sample comprising circulating cell-free nucleic acids, wherein the subject has been administered a therapeutic regimen and wherein the circulating cell-free nucleic acids are mRNA or DNA from normal tissue and cancer tissue, wherein the detecting comprises conducting a sequencing reaction on the circulating cell-free nucleic acids;
b. detecting a second quantity of the cell-free nucleic acids from the cancer tissue in a second biological sample obtained from the subject at a second point in time, wherein the second point of time is within a six-month period after the first biological samples are obtained from the subject, wherein the detecting comprises conducting a sequencing reaction on the circulating cell-free nucleic acids; and
c. adjusting the therapeutic regimen to be administered to the subject based on the first and second quantities, wherein the therapeutic regimen is increased if the second quantity of nucleic acids is 2.5-fold greater than the first quantity of nucleic acids.

Further disclosed herein, in some embodiments, is a method of differential detection of whole genomes, or unique regions thereof, in a biological sample comprising a mixture of genetic material from different genomic sources, the method comprising the steps of: (a) isolating nucleic acid from the biological sample comprising a mixture of genetic material from different genomic sources to obtain a heterogeneous nucleic acid sample; (b) directly sequencing the heterogeneous nucleic acid sample without diluting or distributing the sample into discrete sub-samples or individual molecules; (c) counting the number of unique sequences in the heterogeneous nucleic acid sample; and (d) conducting an analysis that compares the ratios of unique sequences to determine the relative amounts of the different genomes in the biological sample. In some instances, the unique region of the genome comprises one or more variable number tandem repeats (VNTRs), short tandem repeat (STRs), SNP patterns, hypervariable regions, minisatellites, dinucleotide repeats, trinucleotide repeats, tetranucleotide repeats, and simple sequence repeats. In some instances, the sequencing step is performed using long-read sequencing technology. In some instances, the long-read sequencing technology is selected from the group consisting of: the SMRT™ sequencing system, the SOLiD™ sequencing system, the SOLEXA™ sequencing system, the Ion Torrent™ sequencing system, or the Genome Sequencer FLX system. In some instances, the biological sample is blood, a blood fraction, saliva, sputum, urine, semen, transvaginal fluid, cerebrospinal fluid, stool, a cell or a tissue biopsy. In some instances, the blood is peripheral blood derived from a subject diagnosed with or suspected of having cancer, or a fraction thereof. In some instances, the different genomic sources are selected from cancerous cell and a non-cancerous cell. In some instances, the cancer is prostate, breast, ovarian, lung, colon, pancreatic, or skin cancer.

Disclosed herein, in some instances, is a method of treating a subject, the method comprising the steps of: (a) administering a therapeutic regimen to the subject; (b) obtaining a biological sample from the subject and detecting a quantity of nucleic acid from at least one different genomic source within the sample, wherein the at least one different genomic source is different from the subject; and (c) adjusting the therapeutic regimen administered to the subject based on the amount of nucleic acids from the at least one different genomic source, wherein the therapeutic regimen is increased if the percentage of nucleic acids from the at least one different genomic source is greater than 0.5% of the total nucleic acids in the biological sample. In some instances, the percentage of nucleic acids from the at least one different genomic source is less than 1.5% of the total nucleic acids in the biological sample. In some instances, the percentage of nucleic acids from the at least one different genomic source is greater than 1% of the total nucleic acids in the biological sample. In some instances, the subject is a recipient of a heart transplant. In some instances, the biological sample is blood, a blood fraction, saliva, sputum, urine, semen, transvaginal fluid, cerebrospinal fluid, stool, a cell or a tissue biopsy. In some instances, the biological sample is blood. In some instances, the blood is peripheral blood derived from a subject diagnosed with or suspected of having cancer, or a fraction thereof. In some instances, the subject is afflicted with cancer. In some instances, the cancer is prostate, breast, ovarian, lung, colon, pancreatic, or skin cancer. In some instances, the therapeutic regimen is an immune suppression regimen. In some instances, the therapeutic regimen is reduced by at least 50%. In some instances, the immune suppression regimen comprises administering to the subject a glucocorticoid, a cytostatic agent, an anti-metabolite, an antibody, or drugs acting on immunophilins to the subject. In some instances, the immune suppression regimen comprises administering to the subject an anti-IL2 antibody. In some instances, the immune suppression regimen comprises administering to the subject cyclophilin, mycophenolate, basiliximab, daclizumab, tacrolimus, sirolimus, sacrolimus, interferon, opioid, TNF-α (tumor necrosis factor-alpha) binding protein, fingolimod or myriocin. In some instances, the immune suppression regimen comprises administering to the subject CellCept, ProGraf, Simulect, Zenapax, Rapamune, or Nulojix. In some instances, the therapeutic regimen is a chemotherapeutic regimen, a radiation therapy regimen, a monoclonal antibody regimen, an anti-angiogenic regimen, an oligonucleotide therapeutic regimen, or any combination thereof. In some instances, the oligonucleotide therapeutic regimen comprises the administration of an antisense oligonucleotide, miRNA, siRNA, aptamer, or RNA-based therapeutic to the subject. In some instances, the method further comprises sequencing the nucleic acids. In some instances, the sequencing is performed using long-read sequencing technology. In some instances, the long-read sequencing technology is selected from the group consisting of: the SMRT™ sequencing system, the SOLiD™ sequencing system, the SOLEXA™ sequencing system, the Ion Torrent™ sequencing system, or the Genome Sequencer FLX system. In some instances, the method further comprises the steps of counting the number of unique sequences of nucleic acids; and conducting an analysis that compares the ratios of unique sequences to determine the relative amounts of the different genomes in the biological sample. In some instances, the unique region of the genome comprises one or more variable number tandem repeats (VNTRs), short tandem repeat (STRs), SNP patterns, hypervariable regions, minisatellites, dinucleotide repeats, trinucleotide repeats, tetranucleotide repeats, and simple sequence repeats.

Further disclosed herein, in some instances, is a method of treating a subject, the method comprising the steps of: (a) administering a therapeutic regimen to the subject; (b) at a first point of time, obtaining a first biological sample from the subject; (c) detecting a first quantity of nucleic acids from at least one different genomic source within the first biological sample, wherein the at least one different genomic source is different from the subject; (d) at a second point of time, obtaining a second biological sample from the subject at a point of time wherein a transplant rejection is detectable by a biopsy and wherein the second point of time is within a three-month period after the obtaining of the first biological sample from the subject; (e) detecting a second quantity of the nucleic acids from the at least one genomic source within the second biological sample; and (f) adjusting the therapeutic regimen administered to the subject based on the first and second quantities, wherein the therapeutic regimen is increased if the second quantity of nucleic acids is greater than 2.5-fold higher than the first quantity of nucleic acids. In some instances, the biological sample is blood, a blood fraction, saliva, sputum, urine, semen, transvaginal fluid, cerebrospinal fluid, stool, a cell or a tissue biopsy. In some instances, the biological sample is blood. In some instances, the blood is peripheral blood derived from a subject diagnosed with or suspected of having cancer, or a fraction thereof. In some instances, the subject is afflicted with cancer. In some instances, the cancer is prostate, breast, ovarian, lung, colon, pancreatic, or skin cancer. In some instances, the therapeutic regimen is an immune suppression regimen. In some instances, the therapeutic regimen is reduced by at least 50%. In some instances, the immune suppression regimen comprises administering to the subject a glucocorticoid, a cytostatic agent, an anti-metabolite, an antibody, or drugs acting on immunophilins to the subject. In some instances, the immune suppression regimen comprises administering to the subject an anti-IL2 antibody. In some instances, the immune suppression regimen comprises administering to the subject cyclophilin, mycophenolate, basiliximab, daclizumab, tacrolimus, sirolimus, sacrolimus, interferon, opioid, TNF-α (tumor necrosis factor-alpha) binding protein, fingolimod or myriocin. In some instances, the immune suppression regimen comprises administering to the subject CellCept, ProGraf, Simulect, Zenapax, Rapamune, or Nulojix. In some instances, the therapeutic regimen is a chemotherapeutic regimen, a radiation therapy regimen, a monoclonal antibody regimen, an anti-angiogenic regimen, an oligonucleotide therapeutic regimen, or any combination thereof. In some instances, the oligonucleotide therapeutic regimen comprises the administration of an antisense oligonucleotide, miRNA, siRNA, aptamer, or RNA-based therapeutic to the subject. In some instances, the method further comprises sequencing the nucleic acids. In some instances, the sequencing is performed using long-read sequencing technology. In some instances, the long-read sequencing technology is selected from the group consisting of: the SMRT™ sequencing system, the SOLiD™ sequencing system, the SOLEXA™ sequencing system, the Ion Torrent™ sequencing system, or the Genome Sequencer FLX system. In some instances, the method further comprises the steps of counting the number of unique sequences of nucleic acids; and conducting an analysis that compares the ratios of unique sequences to determine the relative amounts of the different genomes in the biological sample. In some instances, the unique region of the genome comprises one or more variable number tandem repeats (VNTRs), short tandem repeat (STRs), SNP patterns, hypervariable regions, minisatellites, dinucleotide repeats, trinucleotide repeats, tetranucleotide repeats, and simple sequence repeats.

Further disclosed herein, in some instances, is a method of treating a subject, the method comprising the steps of (a) administering a therapeutic regimen to the subject; (b) isolating nucleic acid from a biological sample obtained from the subject and detecting within the biological sample a quantity of nucleic acids from at least one different genomic source, wherein the at least one different genomic source is different from the subject; and (c) adjusting the therapeutic regimen administered to the subject based on the amount of nucleic acids from different genomic sources detected in said biological sample, wherein the therapeutic regimen is reduced or stopped if the percentage of nucleic acids from the at least one different genomic source is less than 1% of the total nucleic acids in said biological sample. In some instances, the percentage of nucleic acids from the at least one different genomic source is less than 0.5% of the total nucleic acids in said biological sample. In some instances, the biological sample is blood, a blood fraction, saliva, sputum, urine, semen, transvaginal fluid, cerebrospinal fluid, stool, a cell or a tissue biopsy. In some instances, the biological sample is blood. In some instances, the blood is peripheral blood derived from a subject diagnosed with or suspected of having cancer, or a fraction thereof. In some instances, the subject is a transplant recipient or afflicted with cancer. In some instances, the cancer is prostate, breast, ovarian, lung, colon, pancreatic, or skin cancer. In some instances, the therapeutic regimen is an immune suppression regimen. In some instances, the therapeutic regimen is reduced by at least 50%. In some instances, the immune suppression regimen comprises administering to the subject a glucocorticoid, a cytostatic agent, an anti-metabolite, an antibody, or drugs acting on immunophilins to the subject. In some instances, the immune suppression regimen comprises administering to the subject an anti-IL2 antibody. In some instances, the immune suppression regimen comprises administering to the subject cyclophilin, mycophenolate, basiliximab, daclizumab, tacrolimus, sirolimus, sacrolimus, interferon, opioid, TNF-α (tumor necrosis factor-alpha) binding protein, fingolimod or myriocin. In some instances, the immune suppression regimen comprises administering to the subject CellCept, ProGraf, Simulect, Zenapax, Rapamune, or Nulojix. In some instances, the therapeutic regimen is a chemotherapeutic regimen, a radiation therapy regimen, a monoclonal antibody regimen, an anti-angiogenic regimen, an oligonucleotide therapeutic regimen, or any combination thereof. In some instances, the oligonucleotide therapeutic regimen comprises the administration of an antisense oligonucleotide, miRNA, siRNA, aptamer, or RNA-based therapeutic to the subject. In some instances, the method further comprises sequencing the nucleic acids. In some instances, the sequencing is performed using long-read sequencing technology. In some instances, the long-read sequencing technology is selected from the group consisting of: the SMRT™ sequencing system, the SOLiD™ sequencing system, the SOLEXA™ sequencing system, the Ion Torrent™ sequencing system, or the Genome Sequencer FLX system.

Further disclosed herein, in some instances, is a method of monitoring the immune system in a subject, said method comprising the steps of (a) administering a therapeutic regimen to the subject; (b) at a first point of time, obtaining a first biological sample from the subject and detecting a first quantity of nucleic acid from at least one different genomic source within the biological sample, wherein the at least one different genomic source is different from the subject; (c) at a second point of time, obtaining a second biological sample from the subject at a point of time within three months after the first point of time; (d) detecting a second quantity of nucleic acids from the at least one genomic source within the second biological sample; and (e) adjusting the therapeutic regimen administered to the subject based on the first and second quantities of nucleic acids, wherein the therapeutic regimen is increased if the second quantity of nucleic acids is greater than five-fold higher than the first quantity of nucleic acids. In some instances, the biological sample is blood, a blood fraction, saliva, sputum, urine, semen, transvaginal fluid, cerebrospinal fluid, stool, a cell or a tissue biopsy. In some instances, the biological sample is blood. In some instances, the blood is peripheral blood derived from a subject diagnosed with or suspected of having cancer, or a fraction thereof. The subject may be afflicted with cancer. In some instances, the cancer is prostate, breast, ovarian, lung, colon, pancreatic, or skin cancer. In some instances, the therapeutic regimen is an immune suppression regimen. In some instances, the therapeutic regimen is reduced by at least 50%. In some instances, the immune suppression regimen comprises administering to the subject a glucocorticoid, a cytostatic agent, an anti-metabolite, an antibody, or drugs acting on immunophilins to the subject. In some instances, the immune suppression regimen comprises administering to the subject an anti-IL2 antibody. In some instances, the immune suppression regimen comprises administering to the subject cyclophilin, mycophenolate, basiliximab, daclizumab, tacrolimus, sirolimus, sacrolimus, interferon, opioid, TNF-α (tumor necrosis factor-alpha) binding protein, fingolimod or myriocin. In some instances, the immune suppression regimen comprises administering to the subject CellCept, ProGraf, Simulect, Zenapax, Rapamune, or Nulojix. In some instances, the therapeutic regimen is a chemotherapeutic regimen, a radiation therapy regimen, a monoclonal antibody regimen, an anti-angiogenic regimen, an oligonucleotide therapeutic regimen, or any combination thereof. In some instances, the oligonucleotide therapeutic regimen comprises the administration of an antisense oligonucleotide, miRNA, siRNA, aptamer, or RNA-based therapeutic to the subject. In some instances, the method further comprises sequencing the nucleic acids. In some instances, the sequencing is performed using long-read sequencing technology. In some instances, the long-read sequencing technology is selected from the group consisting of: the SMRT™ sequencing system, the SOLiD™ sequencing system, the SOLEXA™ sequencing system, the Ion Torrent™ sequencing system, or the Genome Sequencer FLX system.

Additional aspects and advantages of the present disclosure will become readily apparent to those skilled in this art from the following detailed description, wherein only illustrative instances of the present disclosure are shown and described. As will be realized, the present disclosure is capable of other and different instances, and its several details are capable of modifications in various obvious respects, all without departing from the disclosure. Accordingly, the drawings and description are to be regarded as illustrative in nature, and not as restrictive.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative instances, in which the principles of the invention are utilized, and the accompanying drawings of which:
**FIGURE 1** Illustration of a workflow for detecting molecules within a heterogeneous sample
**FIGURE 2** Illustration of a workflow for detecting foreign molecules

### DETAILED DESCRIPTION OF THE INVENTION

### I. Overview

This disclosure provides methods, compositions, and systems for detecting molecules (e.g., nucleic acids, proteins, etc.) in a heterogeneous sample, such as a sample comprising nucleic acids derived from at least two different genomic sources. The heterogeneous sample may be a biological sample obtained from a subject and may comprise both the subject's molecules and foreign molecules (e.g., nucleic acids, proteins, etc.) that originated from donor tissue, a pathogen, a fetus, or other source. However, in some cases, the so-called foreign molecules are derived from the subject's own tissue that has transformed in some way-such as by becoming cancerous, or experiencing cellular death (e.g., by necrosis or apoptosis). In such cases, the heterogeneous sample may comprise molecules derived from the subject's healthy tissue as well as molecules derived from tissue that has undergone such change or transformation.

This disclosure is particularly useful for the differential diagnosis of a condition. For example, often the origin of a graft injury experienced by an organ transplant recipient is difficult to determine. A graft injury can also be caused by more than one factor. The present disclosure can enable detection, approximation, or identification of the cause (or multiple causes) of the injury. It also discloses methods of distinguishing between different causes. There are many potential causes of a graft injury including, but not limited to: (1) an immune-mediated rejection of the transplanted tissue and (2) a pathogenic infection. This disclosure provides methods of evaluating a heterogeneous sample in order to evaluate the level of necrosis or apoptosis in the transplanted tissue (or surrounding tissue). The relative levels of necrosis and apoptosis can then be used to assess whether the injury is due to a pathogenic infection (which may correlate with higher levels of necrotic tissue) or a cellular immune response (which may correlate with higher levels of apoptotic tissue). The present disclosure also provides therapeutic regimens, diagnostics, prognostics, and methods of monitoring a condition.

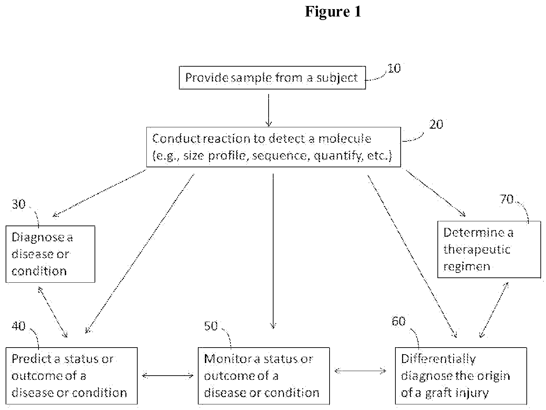

**Figure 1** provides a general overview of the flow of many of the methods provided herein. Generally, the method comprises providing a sample from a subject (10), conducting a reaction to detect a molecule (20), and then diagnosing a disease or condition (30), predicting the status or outcome of a disease or condition (40), monitoring the status or outcome of a disease or condition (50), differentially diagnosing the origin of a graft injury (60), or determining a therapeutic regimen (70). Different combinations of steps can be used, and the steps can be performed in different orders as well. Also provided are methods for detecting, monitoring, and/or measuring whole genomes, or unique regions thereof, within the heterogeneous sample. The genomes (or genotypic patterns) may derive from a subject or from a foreign source.

In some instances, the methods further comprise the use of a computer, computer software, and/or algorithm for analyzing one or more molecules in the sample. In other instances, the methods further comprise generating a report.

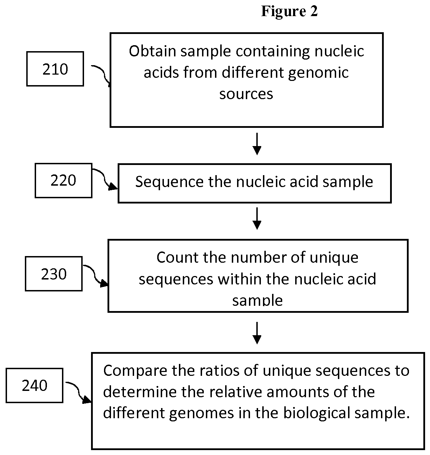

**Figure 2** outlines some additional embodiments of the methods provided herein. The methods may generally comprise: (a) obtaining a sample containing nucleic acids from different genomic sources (110); (b) optionally, sequencing the nucleic acids, e.g., by long-read sequencing (120); (c) optionally, counting the number of unique sequences within the nucleic acid sample (e.g., via sequence reads) (130); and (d) optionally, analyzing (e.g., comparing) the ratios of unique sequences to determine the relative amounts of the different genomes in the biological sample (140). Different combinations of steps can be used, and the steps can be performed in different orders as well, or combined with steps described herein related to other methods.

The methods, compositions, and systems of the disclosure may be especially useful for noninvasive detection of organ rejection in a transplant recipient, cancer in a subject, fetal genetic disorders in a fetus (via the maternal blood), and infection by foreign pathogens. The methods provided herein are also useful for the detection of single nucleotide polymorphisms (SNPs), as well as the detection of any genomic instability, such as a point mutation or an aneuploidy (e.g., trisomy, monosomy, duplication, deletion, addition, rearrangement, translocation, or inversion) within a foreign and/or host genome.

### a. Cancer

This disclosure provides highly sensitive, non-invasive diagnostics for the detection, monitoring or prognosis of cancer using partial or whole genome analysis of circulating nucleic acids derived from tumors as compared to the patient's genome. In some instances, the presence of sequences differing from a patient's normal genotype can be used to detect disease. In cancer, genetic variations such as gene mutations or copy number changes can be predictive of the advance of the disease.

This disclosure provides methods for the detection, monitoring and/or prognosis of cancer in a subject. In some instances, the method comprises detection and/or quantifying a foreign molecule. In some instances, the foreign molecule is a cell-free nucleic acid derived from necrotic or apoptotic cells from tumor circulating within the subject's blood. Methods of evaluating whether a circulating nucleic acid derived from necrotic, apoptotic or normal tissue are provided herein in other sections. Such methods can also be used to detect or monitor necrotic or apoptotic tissue associated with cancer. For example, the method comprises detecting nucleic acids associated with necrotic tissue that are derived from cancerous tissue. In some instances, the method comprises detecting nucleic acids associated with apoptotic tissue that are derived from cancerous tissue. The method may further comprise predicting, evaluating, monitoring, diagnosing, or prognosing the existence of, stage of, or risk of, cancer in the subject based on the level of such nucleic acids (e.g., necrotic nucleic acids or apoptotic nucleic acids). The method may also comprise calculating a Death Mode Ratio, as described herein, to evaluate the relative level of apoptosis or necrosis. The method may also comprise comparing the level of nucleic acids associated with apoptotic tissue with either or both: (a) a healthy subject who does not have cancer and/or (b) a subject known to have cancer. Similarly, the method may comprise calculating a control Death Mode Ratio, as described further herein in other sections.

The method may further comprise evaluating circulating nucleic acids, such as those derived from cancerous cells, for certain hallmark characteristics of disease, such as cancer. This information can be used with, or in place of, information related to apoptosis or necrosis. For example, the circulating nucleic acids can be evaluated for one or more of the following signs of cancer: mutations in oncogenes, microsatellite alterations, and/or viral genomic sequences (which are relevant to cancers caused by viral pathogens such as the human papilloma virus).

Examples of tumor-associated circulating nucleic acids include, but are not limited to, those derived from prostate, breast, ovarian, uterine, cervical, lung, colon, uterine, pancreatic, bladder, brain, liver, kidney, and skin cancer. The disclosure further provides methods for monitoring response to radiation treatment and/or chemotherapeutic drugs, and monitoring cancer remission and recurrence.

In some cases, the heterogeneous samples are from a subject suffering from a cancer and heterogeneous sample comprises foreign molecules derived from a cancerous cell or tumor and molecules derived from a non-cancerous cell. The sample may comprise malignant tissue, benign tissue, or a mixture thereof. The cancer may be a recurrent and/or refractory cancer. Examples of cancers include, but are not limited to, sarcomas, carcinomas, lymphomas or leukemias.

Sarcomas are cancers of the bone, cartilage, fat, muscle, blood vessels, or other connective or supportive tissue. Sarcomas include, but are not limited to, bone cancer, fibrosarcoma, chondrosarcoma, Ewing's sarcoma, malignant hemangioendothelioma, malignant schwannoma, bilateral vestibular schwannoma, osteosarcoma, soft tissue sarcomas (e.g. alveolar soft part sarcoma, angiosarcoma, cystosarcoma phylloides, dermatofibrosarcoma, desmoid tumor, epithelioid sarcoma, extraskeletal osteosarcoma, fibrosarcoma, hemangiopericytoma, hemangiosarcoma, Kaposi's sarcoma, leiomyosarcoma, liposarcoma, lymphangiosarcoma, lymphosarcoma, malignant fibrous histiocytoma, neurofibrosarcoma, rhabdomyosarcoma, and synovial sarcoma).

Carcinomas are cancers that begin in the epithelial cells, which are cells that cover the surface of the body, produce hormones, and make up glands. By way of non-limiting example, carcinomas include breast cancer, pancreatic cancer, lung cancer, colon cancer, colorectal cancer, rectal cancer, kidney cancer, bladder cancer, stomach cancer, prostate cancer, liver cancer, ovarian cancer, brain cancer, vaginal cancer, vulvar cancer, uterine cancer, oral cancer, penile cancer, testicular cancer, esophageal cancer, skin cancer, cancer of the fallopian tubes, head and neck cancer, gastrointestinal stromal cancer, adenocarcinoma, cutaneous or intraocular melanoma, cancer of the anal region, cancer of the small intestine, cancer of the endocrine system, cancer of the thyroid gland, cancer of the parathyroid gland, cancer of the adrenal gland, cancer of the urethra, cancer of the renal pelvis, cancer of the ureter, cancer of the endometrium, cancer of the cervix, cancer of the pituitary gland, neoplasms of the central nervous system (CNS), primary CNS lymphoma, brain stem glioma, and spinal axis tumors. In some instances, the cancer is a skin cancer, such as a basal cell carcinoma, squamous, melanoma, nonmelanoma, or actinic (solar) keratosis.

In some instances, the cancer is a lung cancer. Lung cancer can start in the airways that branch off the trachea to supply the lungs (bronchi) or the small air sacs of the lung (the alveoli). Lung cancers include non-small cell lung carcinoma (NSCLC), small cell lung carcinoma, and mesotheliomia. Examples of NSCLC include squamous cell carcinoma, adenocarcinoma, and large cell carcinoma. The mesothelioma may be a cancerous tumor of the lining of the lung and chest cavitity (pleura) or lining of the abdomen (peritoneum). The mesothelioma may be due to asbestos exposure. The cancer may be a brain cancer, such as a glioblastoma.

Alternatively, the cancer may be a central nervous system (CNS) tumor. CNS tumors may be classified as gliomas or nongliomas. The glioma may be malignant glioma, high grade glioma, diffuse intrinsic pontine glioma. Examples of gliomas include astrocytomas, oligodendrogliomas (or mixtures of oligodendroglioma and astocytoma elements), and ependymomas. Astrocytomas include, but are not limited to, low-grade astrocytomas, anaplastic astrocytomas, glioblastoma multiforme, pilocytic astrocytoma, pleomorphic xanthoastrocytoma, and subependymal giant cell astrocytoma. Oligodendrogliomas include low-grade oligodendrogliomas (or oligoastrocytomas) and anaplastic oligodendriogliomas. Nongliomas include meningiomas, pituitary adenomas, primary CNS lymphomas, and medulloblastomas. In some instances, the cancer is a meningioma.

The leukemia may be an acute lymphocytic leukemia, acute myelocytic leukemia, chronic lymphocytic leukemia, or chronic myelocytic leukemia. Additional types of leukemias include hairy cell leukemia, chronic myelomonocytic leukemia, and juvenile myelomonocytic leukemia.

Lymphomas are cancers of the lymphocytes and may develop from either B or T lymphocytes. The two major types of lymphoma are Hodgkin's lymphoma, previously known as Hodgkin's disease, and non-Hodgkin's lymphoma. Hodgkin's lymphoma is marked by the presence of the Reed-Sternberg cell. Non-Hodgkin's lymphomas are all lymphomas which are not Hodgkin's lymphoma. Non-Hodgkin lymphomas may be indolent lymphomas and aggressive lymphomas. Non-Hodgkin's lymphomas include, but are not limited to, diffuse large B cell lymphoma, follicular lymphoma, mucosa-associated lymphatic tissue lymphoma (MALT), small cell lymphocytic lymphoma, mantle cell lymphoma, Burkitt's lymphoma, mediastinal large B cell lymphoma, Waldenström macroglobulinemia, nodal marginal zone B cell lymphoma (NMZL), splenic marginal zone lymphoma (SMZL), extranodal marginal zone B cell lymphoma, intravascular large B cell lymphoma, primary effusion lymphoma, and lymphomatoid granulomatosis.

Detection of foreign molecules (e.g., cancer-derived molecules) in a subject suffering from cancer may be used in the diagnosis, prediction, or monitoring of a status or outcome of a cancer. For example, diagnosing, predicting, or monitoring a status or outcome of a cancer may comprise diagnosing or detecting a cancer, cancer metastasis, or stage of a cancer. In other instances, diagnosing, predicting, or monitoring a status or outcome of a cancer may comprise predicting the risk of cancer recurrence. In some cases, diagnosing, predicting, or monitoring a status or outcome of a cancer may comprise predicting mortality or morbidity. The methods provided herein may comprise treating a cancer or preventing a cancer progression. In addition, diagnosing, predicting, or monitoring a status or outcome of a cancer may comprise identifying or predicting responders to an anti-cancer therapy.

In some instances, the method comprises determining a therapeutic regimen. Determining a therapeutic regimen may comprise administering an anti-cancer therapy. Alternatively, determining a therapeutic regimen may comprise modifying, recommending, continuing or discontinuing an anti-cancer regimen. An anti-cancer regimen may comprise one or more anti-cancer therapies. Examples of anti-cancer therapies include surgery, chemotherapy, radiation therapy, immunotherapy/biological therapy, photodynamic therapy.

Surgical oncology uses surgical methods to diagnose, stage, and treat cancer, and to relieve certain cancer-related symptoms. Surgery may be used to remove the tumor (e.g., excisions, resections, debulking surgery), reconstruct a part of the body (e.g., restorative surgery), and/or to relieve symptoms such as pain (e.g., palliative surgery). Surgery may also include cryosurgery. Cryosurgery (also called cryotherapy) may use extreme cold produced by liquid nitrogen (or argon gas) to destroy abnormal tissue. Cryosurgery can be used to treat external tumors, such as those on the skin. For external tumors, liquid nitrogen can be applied directly to the cancer cells with a cotton swab or spraying device. Cryosurgery may also be used to treat tumors inside the body (internal tumors and tumors in the bone). For internal tumors, liquid nitrogen or argon gas may be circulated through a hollow instrument called a cryoprobe, which is placed in contact with the tumor. An ultrasound or MRI may be used to guide the cryoprobe and monitor the freezing of the cells, thus limiting damage to nearby healthy tissue. A ball of ice crystals may form around the probe, freezing nearby cells. Sometimes more than one probe is used to deliver the liquid nitrogen to various parts of the tumor. The probes may be put into the tumor during surgery or through the skin (percutaneously). After cryosurgery, the frozen tissue thaws and may be naturally absorbed by the body (for internal tumors), or may dissolve and form a scab (for external tumors).

Chemotherapeutic agents may also be used for the treatment of cancer. Examples of chemotherapeutic agents include alkylating agents, anti-metabolites, plant alkaloids and terpenoids, vinca alkaloids, podophyllotoxin, taxanes, topoisomerase inhibitors, and cytotoxic antibiotics. Cisplatin, carboplatin, and oxaliplatin are examples of alkylating agents. Other alkylating agents include mechlorethamine, cyclophosphamide, chlorambucil, ifosfamide. Alkylating agents may impair cell function by forming covalent bonds with the amino, carboxyl, sulfhydryl, and phosphate groups in biologically important molecules. Alternatively, alkylating agents may chemically modify a cell's DNA.

Anti-metabolites are another example of chemotherapeutic agents. Anti-metabolites may masquerade as purines or pyrimidines and may prevent purines and pyrimidines from becoming incorporated in to DNA during the "S" phase (of the cell cycle), thereby stopping normal development and division. Antimetabolites may also affect RNA synthesis. Examples of metabolites include azathioprine and mercaptopurine.

Alkaloids may be derived from plants, block cell division, and may also be used for the treatment of cancer. Alkaloids may prevent microtubule function. Examples of alkaloids are vinca alkaloids and taxanes. Vinca alkaloids may bind to specific sites on tubulin and inhibit the assembly of tubulin into microtubules (M phase of the cell cycle). The vinca alkaloids may be derived from the Madagascar periwinkle, *Catharanthus roseus* (formerly known as *Vinca rosea*). Examples of vinca alkaloids include, but are not limited to, vincristine, vinblastine, vinorelbine, or vindesine. Taxanes are diterpenes produced by the plants of the genus *Taxus* (yews). Taxanes may be derived from natural sources or synthesized artificially. Taxanes include paclitaxel (Taxol) and docetaxel (Taxotere). Taxanes may disrupt microtubule function. Microtubules are essential to cell division, and taxanes may stabilize GDP-bound tubulin in the microtubule, thereby inhibiting the process of cell division. Thus, in essence, taxanes may be mitotic inhibitors. Taxanes may also be radiosensitizing and often contain numerous chiral centers.

Alternative chemotherapeutic agents include podophyllotoxin and warfarin (coumadin, dicoumarol). Podophyllotoxin is a plant-derived compound that may help with digestion and may be used to produce cytostatic drugs such as etoposide and teniposide. They may prevent the cell from entering the G1 phase (the start of DNA replication) and the replication of DNA (the S phase). Warfarin is a synthetic derivative of dicoumarol, a 4-hydroxycoumarin-derived mycotoxin anticoagulant.

Topoisomerases are essential enzymes that maintain the topology of DNA. Inhibition of type I or type II topoisomerases may interfere with both transcription and replication of DNA by upsetting proper DNA supercoiling. Some chemotherapeutic agents may inhibit topoisomerases. For example, some type I topoisomerase inhibitors include camptothecins: irinotecan and topotecan. Examples of type II inhibitors include amsacrine, etoposide, etoposide phosphate, and teniposide. Alternatively, the anti-cancer agent comprises a proteasome inhibitor. Examples of proteasome inhibitors include bortezomib, disulfiram, epigallocatechin-3-gallage, salinosporamide A, carfilzomib, ONX912, CEP-18770, and MLN9708.

Another example of chemotherapeutic agents is cytotoxic antibiotics. Cytotoxic antibiotics are a group of antibiotics that are used for the treatment of cancer because they may interfere with DNA replication and/or protein synthesis. Cytotoxic antiobiotics include, but are not limited to, actinomycin, anthracyclines, doxorubicin, daunorubicin, valrubicin, idarubicin, epirubicin, bleomycin, plicamycin, and mitomycin.

In some instances, the anti-cancer treatment may comprise radiation therapy. Radiation can come from a machine outside the body (external-beam radiation therapy) or from radioactive material placed in the body near cancer cells (internal radiation therapy, more commonly called brachytherapy). Systemic radiation therapy uses a radioactive substance, given by mouth or into a vein that travels in the blood to tissues throughout the body.

External-beam radiation therapy may be delivered in the form of photon beams (either x-rays or gamma rays). A photon is the basic unit of light and other forms of electromagnetic radiation. An example of external-beam radiation therapy is called 3-dimensional conformal radiation therapy (3D-CRT). 3D-CRT may use computer software and advanced treatment machines to deliver radiation to very precisely shaped target areas. Many other methods of external-beam radiation therapy are currently being tested and used in cancer treatment. These methods include, but are not limited to, intensity-modulated radiation therapy (IMRT), image-guided radiation therapy (IGRT), Stereotactic radiosurgery (SRS), Stereotactic body radiation therapy (SBRT), and proton therapy.

Intensity-modulated radiation therapy (IMRT) is an example of external-beam radiation and may use hundreds of tiny radiation beam-shaping devices, called collimators, to deliver a single dose of radiation. The collimators can be stationary or can move during treatment, allowing the intensity of the radiation beams to change during treatment sessions. This kind of dose modulation allows different areas of a tumor or nearby tissues to receive different doses of radiation. IMRT is planned in reverse (called inverse treatment planning). In inverse treatment planning, the radiation doses to different areas of the tumor and surrounding tissue are planned in advance, and then a high-powered computer program calculates the required number of beams and angles of the radiation treatment. In contrast, during traditional (forward) treatment planning, the number and angles of the radiation beams are chosen in advance and computers calculate how much dose will be delivered from each of the planned beams. The goal of IMRT is to increase the radiation dose to the areas that need it and reduce radiation exposure to specific sensitive areas of surrounding normal tissue.

Another example of external-beam radiation is image-guided radiation therapy (IGRT). In IGRT, repeated imaging scans (CT, MRI, or PET) may be performed during treatment. These imaging scans may be processed by computers to identify changes in a tumor's size and location due to treatment and to allow the position of the patient or the planned radiation dose to be adjusted during treatment as needed. Repeated imaging can increase the accuracy of radiation treatment and may allow reductions in the planned volume of tissue to be treated, thereby decreasing the total radiation dose to normal tissue.

Tomotherapy is a type of image-guided IMRT. A tomotherapy machine is a hybrid between a CT imaging scanner and an external-beam radiation therapy machine. The part of the tomotherapy machine that delivers radiation for both imaging and treatment can rotate completely around the patient in the same manner as a normal CT scanner. Tomotherapy machines can capture CT images of the patient's tumor immediately before treatment sessions, to allow for very precise tumor targeting and sparing of normal tissue.

Stereotactic radiosurgery (SRS) can deliver one or more high doses of radiation to a small tumor. SRS uses extremely accurate image-guided tumor targeting and patient positioning. Therefore, a high dose of radiation can be given without excess damage to normal tissue. SRS can be used to treat small tumors with well-defined edges. It is most commonly used in the treatment of brain or spinal tumors and brain metastases from other cancer types. For the treatment of some brain metastases, patients may receive radiation therapy to the entire brain (called whole-brain radiation therapy) in addition to SRS. SRS requires the use of a head frame or other device to immobilize the patient during treatment to ensure that the high dose of radiation is delivered accurately.

Stereotactic body radiation therapy (SBRT) delivers radiation therapy in fewer sessions, using smaller radiation fields and higher doses than 3D-CRT in most cases. SBRT may treat tumors that lie outside the brain and spinal cord. Because these tumors are more likely to move with the normal motion of the body, and therefore cannot be targeted as accurately as tumors within the brain or spine, SBRT is usually given in more than one dose. SBRT can be used to treat small, isolated tumors, including cancers in the lung and liver. SBRT systems may be known by their brand names, such as the CyberKnife®.

In proton therapy, external-beam radiation therapy may be delivered by proton. Protons are a type of charged particle. Proton beams differ from photon beams mainly in the way they deposit energy in living tissue. Whereas photons deposit energy in small packets all along their path through tissue, protons deposit much of their energy at the end of their path (called the Bragg peak) and deposit less energy along the way. Use of protons may reduce the exposure of normal tissue to radiation, possibly allowing the delivery of higher doses of radiation to a tumor.

Other charged particle beams such as electron beams may be used to irradiate superficial tumors, such as skin cancer or tumors near the surface of the body, but they cannot travel very far through tissue.

Internal radiation therapy (brachytherapy) is radiation delivered from radiation sources (radioactive materials) placed inside or on the body. Several brachytherapy techniques are used in cancer treatment. Interstitial brachytherapy may use a radiation source placed within tumor tissue, such as within a prostate tumor. Intracavitary brachytherapy may use a source placed within a surgical cavity or a body cavity, such as the chest cavity, near a tumor. Episcleral brachytherapy, which may be used to treat melanoma inside the eye, may use a source that is attached to the eye. In brachytherapy, radioactive isotopes can be sealed in tiny pellets or "seeds." These seeds may be placed in patients using delivery devices, such as needles, catheters, or some other type of carrier. As the isotopes decay naturally, they give off radiation that may damage nearby cancer cells. Brachytherapy may be able to deliver higher doses of radiation to some cancers than external-beam radiation therapy while causing less damage to normal tissue.

Brachytherapy can be given as a low-dose-rate or a high-dose-rate treatment. In low-dose-rate treatment, cancer cells receive continuous low-dose radiation from the source over a period of several days. In high-dose-rate treatment, a robotic machine attached to delivery tubes placed inside the body may guide one or more radioactive sources into or near a tumor, and then removes the sources at the end of each treatment session. High-dose-rate treatment can be given in one or more treatment sessions. An example of a high-dose-rate treatment is the MammoSite® system. Brachytherapy may be used to treat patients with breast cancer who have undergone breast-conserving surgery.

The placement of brachytherapy sources can be temporary or permanent. For permament brachytherapy, the sources may be surgically sealed within the body and left there, even after all of the radiation has been given off. In some instances, the remaining material (in which the radioactive isotopes were sealed) does not cause any discomfort or harm to the patient. Permanent brachytherapy is a type of low-dose-rate brachytherapy. For temporary brachytherapy, tubes (catheters) or other carriers are used to deliver the radiation sources, and both the carriers and the radiation sources are removed after treatment. Temporary brachytherapy can be either low-dose-rate or high-dose-rate treatment. Brachytherapy may be used alone or in addition to external-beam radiation therapy to provide a "boost" of radiation to a tumor while sparing surrounding normal tissue.

In systemic radiation therapy, a patient may swallow or receive an injection of a radioactive substance, such as radioactive iodine or a radioactive substance bound to a monoclonal antibody. Radioactive iodine (1311) is a type of systemic radiation therapy commonly used to help treat cancer, such as thyroid cancer. Thyroid cells naturally take up radioactive iodine. For systemic radiation therapy for some other types of cancer, a monoclonal antibody may help target the radioactive substance to the right place. The antibody joined to the radioactive substance travels through the blood, locating and killing tumor cells. For example, the drug ibritumomab tiuxetan (Zevalin®) may be used for the treatment of certain types of B-cell non-Hodgkin lymphoma (NHL). The antibody part of this drug recognizes and binds to a protein found on the surface of B lymphocytes. The combination drug regimen of tositumomab and iodine I131 tositumomab (Bexxar®) may be used for the treatment of certain types of cancer, such as NHL. In this regimen, nonradioactive tositumomab antibodies may be given to patients first, followed by treatment with tositumomab antibodies that have 1311 attached. Tositumomab may recognize and bind to the same protein on B lymphocytes as ibritumomab. The nonradioactive form of the antibody may help protect normal B lymphocytes from being damaged by radiation from 1311.

Some systemic radiation therapy drugs relieve pain from cancer that has spread to the bone (bone metastases). This is a type of palliative radiation therapy. The radioactive drugs samarium-153-lexidronam (Quadramet®) and strontium-89 chloride (Metastron®) are examples of radiopharmaceuticals may be used to treat pain from bone metastases.

Biological therapy (sometimes called immunotherapy, biotherapy, or biological response modifier (BRM) therapy) uses the body's immune system, either directly or indirectly, to fight cancer or to lessen the side effects that may be caused by some cancer treatments. Biological therapies include interferons, interleukins, colony-stimulating factors, monoclonal antibodies, vaccines, gene therapy, and nonspecific immunomodulating agents.

Interferons (IFNs) are types of cytokines that occur naturally in the body. Interferon alpha, interferon beta, and interferon gamma are examples of interferons that may be used in cancer treatment.

Like interferons, interleukins (ILs) are cytokines that occur naturally in the body and can be made in the laboratory. Many interleukins have been identified for the treatment of cancer. For example, interleukin-2 (IL-2 or aldesleukin), interleukin 7, and interleukin 12 have may be used as an anti-cancer treatment. IL-2 may stimulate the growth and activity of many immune cells, such as lymphocytes, that can destroy cancer cells. Interleukins may be used to treat a number of cancers, including leukemia, lymphoma, and brain, colorectal, ovarian, breast, kidney and prostate cancers.

Colony-stimulating factors (CSFs) (sometimes called hematopoietic growth factors) may also be used for the treatment of cancer. Some examples of CSFs include, but are not limited to, G-CSF (filgrastim) and GM-CSF (sargramostim). CSFs may promote the division of bone marrow stem cells and their development into white blood cells, platelets, and red blood cells. Bone marrow is critical to the body's immune system because it is the source of all blood cells. Because anticancer drugs can damage the body's ability to make white blood cells, red blood cells, and platelets, stimulation of the immune system by CSFs may benefit patients undergoing other anti-cancer treatment, thus CSFs may be combined with other anti-cancer therapies, such as chemotherapy. CSFs may be used to treat a large variety of cancers, including lymphoma, leukemia, multiple myeloma, melanoma, and cancers of the brain, lung, esophagus, breast, uterus, ovary, prostate, kidney, colon, and rectum.

Another type of biological therapy includes monoclonal antibodies (MOABs or MoABs). These antibodies may be produced by a single type of cell and may be specific for a particular antigen. To create MOABs, a human cancer cells may be injected into mice. In response, the mouse immune system can make antibodies against these cancer cells. The mouse plasma cells that produce antibodies may be isolated and fused with laboratory-grown cells to create "hybrid" cells called hybridomas. Hybridomas can indefinitely produce large quantities of these pure antibodies, or MOABs. MOABs may be used in cancer treatment in a number of ways. For instance, MOABs that react with specific types of cancer may enhance a patient's immune response to the cancer. MOABs can be programmed to act against cell growth factors, thus interfering with the growth of cancer cells.

MOABs may be linked to other anti-cancer therapies such as chemotherapeutics, radioisotopes (radioactive substances), other biological therapies, or other toxins. When the antibodies latch onto cancer cells, they deliver these anti-cancer therapies directly to the tumor, helping to destroy it. MOABs carrying radioisotopes may also prove useful in diagnosing certain cancers, such as colorectal, ovarian, and prostate.

Rituxan® (rituximab) and Herceptin® (trastuzumab) are examples of MOABs that may be used as a biological therapy. Rituxan may be used for the treatment of non-Hodgkin lymphoma. Herceptin can be used to treat metastatic breast cancer in patients with tumors that produce excess amounts of a protein called HER2. Alternatively, MOABs may be used to treat lymphoma, leukemia, melanoma, and cancers of the brain, breast, lung, kidney, colon, rectum, ovary, prostate, and other areas.

Cancer vaccines are another form of biological therapy. Cancer vaccines may be designed to encourage the patient's immune system to recognize cancer cells. Cancer vaccines may be designed to treat existing cancers (therapeutic vaccines) or to prevent the development of cancer (prophylactic vaccines). Therapeutic vaccines may be injected in a person after cancer is diagnosed. These vaccines may stop the growth of existing tumors, prevent cancer from recurring, or eliminate cancer cells not killed by prior treatments. Cancer vaccines given when the tumor is small may be able to eradicate the cancer. On the other hand, prophylactic vaccines are given to healthy individuals before cancer develops. These vaccines are designed to stimulate the immune system to attack viruses that can cause cancer. By targeting these cancer-causing viruses, development of certain cancers may be prevented. For example, cervarix and gardasil are vaccines to treat human papilloma virus and may prevent cervical cancer. Therapeutic vaccines may be used to treat melanoma, lymphoma, leukemia, and cancers of the brain, breast, lung, kidney, ovary, prostate, pancreas, colon, and rectum. Cancer vaccines can be used in combination with other anti-cancer therapies.

Gene therapy is another example of a biological therapy. Gene therapy may involve introducing genetic material into a person's cells to fight disease. Gene therapy methods may improve a patient's immune response to cancer. For example, a gene may be inserted into an immune cell to enhance its ability to recognize and attack cancer cells. In another approach, cancer cells may be injected with genes that cause the cancer cells to produce cytokines and stimulate the immune system.

In some instances, biological therapy includes nonspecific immunomodulating agents. Nonspecific immunomodulating agents are substances that stimulate or indirectly augment the immune system. Often, these agents target key immune system cells and may cause secondary responses such as increased production of cytokines and immunoglobulins. Two nonspecific immunomodulating agents used in cancer treatment are bacillus Calmette-Guerin (BCG) and levamisole. BCG may be used in the treatment of superficial bladder cancer following surgery. BCG may work by stimulating an inflammatory, and possibly an immune, response. A solution of BCG may be instilled in the bladder. Levamisole is sometimes used along with fluorouracil (5-FU) chemotherapy in the treatment of stage III (Dukes' C) colon cancer following surgery. Levamisole may act to restore depressed immune function.

Photodynamic therapy (PDT) is an anti-cancer treatment that may use a drug, called a photosensitizer or photosensitizing agent, and a particular type of light. When photosensitizers are exposed to a specific wavelength of light, they may produce a form of oxygen that kills nearby cells. A photosensitizer may be activated by light of a specific wavelength. This wavelength determines how far the light can travel into the body. Thus, photosensitizers and wavelengths of light may be used to treat different areas of the body with PDT.

In the first step of PDT for cancer treatment, a photosensitizing agent may be injected into the bloodstream. The agent may be absorbed by cells all over the body but may stay in cancer cells longer than it does in normal cells. Approximately 24 to 72 hours after injection, when most of the agent has left normal cells but remains in cancer cells, the tumor can be exposed to light. The photosensitizer in the tumor can absorb the light and produces an active form of oxygen that destroys nearby cancer cells. In addition to directly killing cancer cells, PDT may shrink or destroy tumors in two other ways. The photosensitizer can damage blood vessels in the tumor, thereby preventing the cancer from receiving necessary nutrients. PDT may also activate the immune system to attack the tumor cells.

The light used for PDT can come from a laser or other sources. Laser light can be directed through fiber optic cables (thin fibers that transmit light) to deliver light to areas inside the body. For example, a fiber optic cable can be inserted through an endoscope (a thin, lighted tube used to look at tissues inside the body) into the lungs or esophagus to treat cancer in these organs. Other light sources include light-emitting diodes (LEDs), which may be used for surface tumors, such as skin cancer. PDT is usually performed as an outpatient procedure. PDT may also be repeated and may be used with other therapies, such as surgery, radiation, or chemotherapy.

Extracorporeal photopheresis (ECP) is a type of PDT in which a machine may be used to collect the patient's blood cells. The patient's blood cells may be treated outside the body with a photosensitizing agent, exposed to light, and then returned to the patient. ECP may be used to help lessen the severity of skin symptoms of cutaneous T-cell lymphoma that has not responded to other therapies. ECP may be used to treat other blood cancers, and may also help reduce rejection after transplants.

Additionally, photosensitizing agent, such as porfimer sodium or Photofrin®, may be used in PDT to treat or relieve the symptoms of esophageal cancer and non-small cell lung cancer. Porfimer sodium may relieve symptoms of esophageal cancer when the cancer obstructs the esophagus or when the cancer cannot be satisfactorily treated with laser therapy alone. Porfimer sodium may be used to treat non-small cell lung cancer in patients for whom the usual treatments are not appropriate, and to relieve symptoms in patients with non-small cell lung cancer that obstructs the airways. Porfimer sodium may also be used for the treatment of precancerous lesions in patients with Barrett esophagus, a condition that can lead to esophageal cancer.

Laser therapy may use high-intensity light to treat cancer and other illnesses. Lasers can be used to shrink or destroy tumors or precancerous growths. Lasers are most commonly used to treat superficial cancers (cancers on the surface of the body or the lining of internal organs) such as basal cell skin cancer and the very early stages of some cancers, such as cervical, penile, vaginal, vulvar, and non-small cell lung cancer.

Lasers may also be used to relieve certain symptoms of cancer, such as bleeding or obstruction. For example, lasers can be used to shrink or destroy a tumor that is blocking a patient's trachea (windpipe) or esophagus. Lasers also can be used to remove colon polyps or tumors that are blocking the colon or stomach.

Laser therapy is often given through a flexible endoscope (a thin, lighted tube used to look at tissues inside the body). The endoscope is fitted with optical fibers (thin fibers that transmit light). It is inserted through an opening in the body, such as the mouth, nose, anus, or vagina. Laser light is then precisely aimed to cut or destroy a tumor.

Laser-induced interstitial thermotherapy (LITT), or interstitial laser photocoagulation, also uses lasers to treat some cancers. LITT is similar to a cancer treatment called hyperthermia, which uses heat to shrink tumors by damaging or killing cancer cells. During LITT, an optical fiber is inserted into a tumor. Laser light at the tip of the fiber raises the temperature of the tumor cells and damages or destroys them. LITT is sometimes used to shrink tumors in the liver.

Laser therapy can be used alone, but most often it is combined with other treatments, such as surgery, chemotherapy, or radiation therapy. In addition, lasers can seal nerve endings to reduce pain after surgery and seal lymph vessels to reduce swelling and limit the spread of tumor cells.

Lasers used to treat cancer may include carbon dioxide (CO2) lasers, argon lasers, and neodymium:yttrium-aluminum-garnet (Nd:YAG) lasers. Each of these can shrink or destroy tumors and can be used with endoscopes. CO2 and argon lasers can cut the skin's surface without going into deeper layers. Thus, they can be used to remove superficial cancers, such as skin cancer. In contrast, the Nd:YAG laser is more commonly applied through an endoscope to treat internal organs, such as the uterus, esophagus, and colon. Nd:YAG laser light can also travel through optical fibers into specific areas of the body during LITT. Argon lasers are often used to activate the drugs used in PDT.

### V. General Therapeutic Diagnoses/Predictions/Regimens

In some instances, the methods, compositions, and systems disclosed here are used to diagnose a disease or condition in a subject. Diagnosing a disease or condition may comprise diagnosing a disease or condition such as cancer. Diagnosing a disease or condition may comprise confirming a preliminary diagnosis. In some cases, diagnosing a disease or condition comprises determining the stage or level of severity of a disease, such as cancer; or otherwise classifying a disease. In a particular embodiment, the methods of the invention provide the capability for sensitive, non-invasive, high throughput screening for diseases or conditions associated with the release of circulating nucleic acids into the bloodstream of a subject. The disease or condition may be a cancer, an organ transplant, a pathogenic infection, or pregnancy. The circulating nucleic acids are foreign nucleic acids and may be from a cancerous cell or tissue, a donor organ, a pathogen, or a fetus. In some instances, the circulating nucleic acids are host-derived nucleic acids and may be from a non-cancerous cell or a subject tissue, organ, or cell.

In other instances, the methods, compositions, and system disclosed herein are used to predict a status or outcome of a disease or condition. Predicting a status or outcome of a disease or condition may comprise predicting the risk of disease or injury. Predicting a status or outcome of a disease or condition may comprise predicting the risk of recurrence. Alternatively, predicting a status or outcome of a disease or condition may comprise predicting mortality or morbidity. In some instances, predicting a status or outcome of a disease or condition comprises identifying or predicting therapeutic responders. In other instances, predicting a status or outcome of a disease or condition comprises predicting risk of drug resistance.

In some instances, predicting a status or outcome of a disease or condition comprises predicting a transplant rejection or risk of a transplant rejection. Alternatively, predicting a status or outcome of a disease or condition comprises predicting the risk of cancer recurrence. Predicting a status or outcome of a disease or condition can also comprise predicting the risk of infection or injury. In some instances, predicting a status or outcome of a disease or condition comprises predicting an effectiveness of a therapeutic regimen or predicting a response to a therapeutic regimen.

Monitoring a status or outcome of a disease or condition may comprise preventing progression of the disease or condition. Alternatively, monitoring a status or outcome of a disease or condition comprises determining an efficacy of a therapeutic drug or regimen. The efficacy of a therapeutic regimen can be determined by detecting foreign molecules before, during and/or after a therapeutic drug or regimen is administered. In some instances, a decrease in foreign molecules is indicative of drug efficacy. For example, if the foreign molecules decrease by at least about 5%, at least about 10%, at least about 20%, at least about 30%, at least about 40%, at least about 50%, at least about 60%, at least about 70%, at least about 80%, at least about 90%, or at least about 95%, then the therapeutic regimen is deemed effective. Alternatively, if the foreign molecules decrease by at least about 20-fold, at least about 15-fold, at least about 10-fold, at least about 7-fold, at least about 5-fold, at least about 4-fold, at least about 3-fold, at least about 2-fold, at least about 1.5-fold, or at least about 1-fold, then the therapeutic regimen is deemed effective. In another example, if the foreign molecules comprise less than about 10%, less than about 7%, less than about 5%, less than about 4%, less than about 3%, less than about 2%, less than about 1.5%, less than about 1%, less than about 0.9%, less than about 0.8%, less than about 0.7%, less than about 0.6%, less than about 0.5%, less than about 0.4%, less than about 0.3%, less than about 0.2%, or less than about 0.1% of the total molecules in the sample, than the drug is deemed effective. Alternatively, an increase in foreign molecules is indicative of drug failure, inefficiency, or refraction. For example, if the foreign molecules of increases by at least about 5%, at least about 10%, at least about 20%, at least about 30%, at least about 40%, at least about 50%, at least about 60%, at least about 70%, at least about 80%, at least about 90%, or at least about 95%, then the therapeutic regimen is deemed a failure or ineffective, or the subject is deemed refractory to the drug. Alternatively, if the foreign molecules increase by at least about 1-fold, at least about 1.5-fold, at least about 2-fold, at least about 3-fold, at least about 4-fold, at least about 5-fold, at least about 7-fold, at least about 10-fold, at least about 15-fold, or at least about 20-fold, then the therapeutic regimen is deemed a failure or ineffective, or the subject is deemed refractory to the drug. In another example, if the foreign molecules comprise greater than about 0.1%, greater than about 0.2%, greater than about 0.3%, greater than about 0.4%, greater than about 0.5%, greater than about 0.6%, greater than about 0.7%, greater than about 0.8%, greater than about 0.9%, greater than about 1%, greater than about 2%, greater than about 3%, greater than about 4%, greater than about 5%, greater than about 7%, greater than about 10%, greater than about 15%, or greater than about 20% of the total molecules in the sample, than the drug is deemed a failure or ineffective, or the subject is deemed refractory to the drug.

In some instances, the quantitative measurement of cell-free nucleic acids found within the various biological samples obtained from the subject, as described herein, is indicative of whether the therapeutic regimen is effective to treat a particular disease or condition (e.g., cancer, transplant rejection, pathogenic infection, or chimerism), or whether it needs to be adjusted to increase efficacy, or to avoid over-administration of harsh or harmful drugs or agents to the subject. In certain embodiments, the therapeutic regimen is increased if the percentage of cell-free nucleic acids from different genomic sources is greater than 1-2% of the total nucleic acids, preferably greater than or equal to 1% of the total nucleic acids, in the biological sample obtained from the subject. In other certain embodiments, the therapeutic regimen is decreased if the percentage of nucleic acids from different genomic sources is less than 1%, 0.9%, 0.8%, 0.7%, 0.6%, 0.5%, 0.4%, 0.3%, 0.2%, or 0.1% of the total nucleic acids in the biological sample.

In certain embodiments, the therapeutic drug is titrated and the percentage of cell-free nucleic acids (e.g., DNA, RNA) from different genomic sources is determined for a plurality of titration points. The percentage of cell-free nucleic acids for the plurality of titration points can be used to inform the proper dose of the therapeutic treatment regimen. The concentration endpoint may be specific to a particular organ, or particular individual, or both.

In some instances, the methods, compositions and systems disclosed herein are used to determine a treatment regimen. Determining a treatment regimen may comprise administering a drug (e.g., immunosuppressive therapy, anti-cancer drug, anti-microbial). Alternatively, determining a treatment regimen may comprise modifying, recommending, or initiating a therapeutic regimen. Modifying a therapeutic regimen comprises continuing, discontinuing, increasing, or decreasing a therapeutic regimen. In some instances, determining a treatment regimen comprises determining an optimal dose and/or optimal dosing schedule based on the presence or absence of foreign molecules. A therapeutic regimen may comprise one or more therapeutic drugs. The therapeutic regimen may comprise at least about 1, at least about 2, at least about 3, at least about 4, at least about 5, at least about 6, at least about 7, at least about 8, at least about 9, or at least about 10 therapeutic drugs.

In some instances, determining a therapeutic regimen comprises determining drug-specific baselines and/or thresholds. The drug-specific baselines and/or thresholds can provide ranges for modifying (e.g., adjusting, maintaining, initiating, or terminating) a therapeutic regimen. In some instances, modifying a therapeutic regimen may comprise increasing or decreasing a dosage of a therapeutic drug based on the presence or absence of foreign molecules. The dosage of therapeutic drug may be increased if the percentage of foreign molecules in the sample increases. The dosage of the therapeutic drug may be increased by at least about 2%, 5%, 7%, 10%, 15%, 20%, 30%, 40%, 50%, 60%, 70%, 80%, 90%, 95%, and 97%. Alternatively, the dosage of the therapeutic drug may be decreased if the percentage of foreign molecules in the sample decreases. The dosage of the therapeutic drug may be decreased by at least about 2%, 5%, 7%, 10%, 15%, 20%, 30%, 40%, 50%, 60%, 70%, 80%, 90%, 95%, and 97%.

In certain cases, the therapeutic regimen is modified if the percentage of nucleic acids from different genomic sources (e.g., foreign nucleic acids) is greater than about 1% of the total molecules (e.g., nucleic acids) in the biological sample obtained from the subject. In some instances, modifying a therapeutic regimen comprises increasing a therapeutic regimen. The therapeutic regimen may be increased if the percentage of nucleic acids from different genomic sources (e.g., foreign nucleic acids) is greater than about 0.1%, greater than about 0.2%, greater than about 0.3%, greater than about 0.4%, greater than about 0.5%, greater than about 0.6%, 0.7%, greater than about 0.8%, greater than about 0.9%, greater than about 1%, greater than about 2%, greater than about 3%, greater than about 4%, greater than about 5%, greater than about 6%, greater than about 7%, greater than about 8%, greater than about 9%, or greater than about 10% of the total molecules (e.g., nucleic acids) in the sample. Alternatively, if the percentage of foreign molecules is greater than about 0.1%, greater than about 0.2%, greater than about 0.3%, greater than about 0.4%, greater than about 0.5%, greater than about 0.6%, greater than about 0.7%, greater than about 0.8%, greater than about 0.9%, greater than about 1%, greater than about 2%, greater than about 3%, greater than about 4%, greater than about 5%, greater than about 6%, greater than about 7%, greater than about 8%, greater than about 9%, or greater than about 10% of the total molecules in the sample, then the therapeutic regimen is administered, increased, or initiated. Alternatively, if the percentage of foreign molecules is greater than about 0.1%, greater than about 0.2%, greater than about 0.3%, greater than about 0.4%, greater than about 0.5%, greater than about 0.6%, greater than about 0.7%, greater than about 0.8%, greater than about 0.9%, greater than about 1%, greater than about 2%, greater than about 3%, greater than about 4%, greater than about 5%, greater than about 6%, greater than about 7%, greater than about 8%, greater than about 9%, or greater than about 10% of the total molecules in the sample, then the frequency of dosage of the therapeutic drug is increased.

In other certain embodiments, the therapeutic regimen is modified if the percentage of nucleic acids from different genomic sources (e.g., foreign nucleic acids) is less than about 4%, 3%, 2%, 1%, 0.9%, 0.8%, 0.7%, 0.6%, 0.5%, 0.4%, 0.3%, 0.2%, or 0.1% of the total molecules (e.g., nucleic acids) in the biological sample obtained from the subject. In some instances, modifying a therapeutic regimen comprises decreasing a therapeutic regimen. The therapeutic regimen may be decreased if the percentage of nucleic acids from different genomic sources (e.g., foreign nucleic acids) is less than about 10%, less than about 7%, less than about 5%, less than about 3%, less than about 2%, less than about 1.5%, less than about 1%, less than about 0.7%, less than about 0.5%, or less than about 0.1% of the total molecules (e.g., nucleic acids) in the sample. Alternatively, if the percentage of foreign molecules is less than about 10%, less than about 7%, less than about 5%, less than about 4%, less than about 3%, less than about 2%, less than about 1%, less than about 0.9%, less than about 0.8%, less than about 0.7%, less than about 0.6%, less than about 0.5%, less than about 0.4%, less than about 0.3%, less than about 0.2%, or less than about 0.1% of the total molecules (e.g, nucleic acids, DNA) in the sample, then the therapeutic regimen is decreased or terminated. Alternatively, if the percentage of foreign molecules is less than about 10%, less than about 7%, less than about 5%, less than about 4%, less than about 3%, less than about 2%, less than about 1%, less than about 0.9%, less than about 0.8%, less than about 0.7%, less than about 0.6%, less than about 0.5%, less than about 0.4%, less than about 0.3%, less than about 0.2%, or less than about 0.1% of the total molecules (e.g., nucleic acids, DNA) in the sample, then the frequency of dosage of the therapeutic drug is decreased.

In some instances, the foreign molecules increase by at least about 30%, 40%, 50%, 60%, 70%, 80%, 90%, 95%, or 100%. In other instances, the foreign molecules increase by at least about 1.5-fold, at least about 2-fold, at least about 2.5-fold, at least about 3-fold, at least about 3.5-fold, at least about 4-fold, at least about 4.5-fold, at least about 5-fold, at least about 6-fold, at least about 7-fold, at least about 8-fold, at least about 9-fold, or at least about 10-fold. Alternatively, the foreign molecules increase by at least about 20-fold, at least about 30-fold, at least about 40-fold, at least about 50-fold, at least about 60-fold, at least about 70-fold, at least about 80-fold, at least about 90-fold, or at least about 100-fold.

In some instances, the foreign molecules decrease by at least about 30%, 40%, 50%, 60%, 70%, 80%, 90%, 95%, or 100%. In other instances, the foreign molecules decrease by at least about 1.5-fold, at least about 2-fold, at least about 2.5-fold, at least about 3-fold, at least about 3.5-fold, at least about 4-fold, at least about 4.5-fold, at least about 5-fold, at least about 6-fold, at least about 7-fold, at least about 8-fold, at least about 9-fold, or at least about 10-fold. Alternatively, the foreign molecules decrease by at least about 20-fold, at least about 30-fold, at least about 40-fold, at least about 50-fold, at least about 60-fold, at least about 70-fold, at least about 80-fold, at least about 90-fold, or at least about 100-fold.

In some instances, determining a therapeutic regimen comprises administering a drug. The method may further comprise administering a test prior to administration of the drug. In some instances, the test comprises detecting the presence or absence of a foreign molecule. The test may comprise a diagnostic assay. For example, the test may comprise a viral detection test to diagnose a viral infection in a subject. Alternatively, the test comprises a bacterial detection test to diagnose a bacterial infection in a subject. In another instance, the test is a genetic test. For example, the subject is a pregnant female and the genetic test is administered to detect a fetal genetic abnormality. If a fetal genetic abnormality is detected, a drug may be administered to the subject.

In some instances, the methods, compositions, and systems disclosed herein are used as a companion diagnostic, whereby molecular assays that measure levels of proteins, nucleic acids, genes or specific mutations are used to provide a specific therapy for an individual's condition by stratifying disease status, selecting the proper medication and tailoring dosages to that patient's specific needs. Alternatively, such methods might be used to monitor the efficacy and/or toxicity of an immunosuppressive therapy administered to a subject suffering from a transplant rejection. The immunosuppressive therapy may be increased, decreased, or terminated based on the results of monitoring. In other instances, a new immunosuppressive therapy may be administered based on the results of the monitoring. Additionally, such methods might be used to assess a patient's risk factor for a number of conditions and tailor individual preventative treatments such as nutritional immunology approaches. Tissue-derived molecular information might be combined with an individual's personal medical history, family history, and data from imaging, and other laboratory tests to develop more effective treatments for a wider variety of conditions.

Determining a therapeutic regimen may comprise administering, modifying, initiating, or terminating a therapeutic regimen. Modifying a therapeutic regimen may comprise increasing or decreasing the dose of a therapeutic drug(s) and/or frequency of dosage of a therapeutic drug(s). Alternatively, modifying a therapeutic regimen can comprise adding or removing one or more therapeutic drugs. In some instances, determining a therapeutic regimen comprises determining the effective dose of a therapeutic drug. Alternatively, determining a therapeutic regimen comprises determining a dosing schedule for a therapeutic drug. The therapeutic regimen may be an anti-cancer regimen, immunosuppressive regimen, anti-pathogenic regimen (e.g., antibacterial, antiviral, antifungal). Alternatively, therapeutic regimen is a chemotherapeutic regimen, a radiation therapy regimen, a monoclonal antibody regimen, an anti-angiogenic regimen, an oligonucleotide therapeutic regimen, or any combination thereof. In some instances, the oligonucleotide therapeutic regimen comprises an antisense oligonucleotide, an miRNA, an siRNA, an aptamer or an RNA-based therapeutic. Often, the therapeutic regimens provided herein have better performance than standard regimens since circulating nucleic acids can serve as a better marker for the diagnosis, prediction, or monitoring of a status or outcome of a disease or condition as compared to standard markers (e.g., serum creatinine levels for kidney function or transaminase levels for liver function).

### VI. Detection of molecules

### Types of Molecules Detected

The methods disclosed herein often comprise conducting a reaction to detect a molecule (e.g., foreign molecule) in a heterogeneous sample from a subject. The method may further comprise detecting a molecule derived from a subject. The molecule (e.g., foreign molecule or subject-derived molecule) may be a biomolecule. Examples of biomolecules include, but are not limited to, a protein, a polypeptide, a peptide, a nucleic acid molecule, a nucleotide, an oligonucleotide, a polynucleotide, a saccharide, a polysaccharide, a cytokine, a growth factor, a morphogen, an antibody, a peptibody, or any fragment thereof. In some instances, the foreign molecule is a nucleic acid molecule or fragment thereof. Additionally, the subject molecule is a nucleic acid molecule or fragment thereof. The nucleic acid molecule may be a DNA molecule, RNA molecule (e.g. mRNA, cRNA or miRNA), and DNA/RNA hybrids. Examples of DNA molecules include, but are not limited to, double-stranded DNA, single-stranded DNA, single-stranded DNA hairpins, cDNA, genomic DNA. The nucleic acid may be an RNA molecule, such as a double-stranded RNA, single-stranded RNA, ncRNA, RNA hairpin, and mRNA. Examples of ncRNA include, but are not limited to, siRNA, miRNA, snoRNA, piRNA, tiRNA, PASR, TASR, aTASR, TSSa-RNA, snRNA, RE-RNA, uaRNA, x-ncRNA, hY RNA, usRNA, snaR, and vtRNA.

The DNA or RNA can be cell-free DNA or cell-free RNA. The DNA or RNA can be circulating, such as circulating within a bodily fluid (e.g., blood, urine). Circulating nucleic acids in bodily fluids such as blood can arise from necrotic or apoptotic cells. In some particular cases, the method comprises detecting or isolating cell-free RNA present in human plasma (Tong, Y.K. Lo, Y.M., Clin Chim Acta, 363, 187-196 (2006)) and cDNA sequencing of transcripts, thereby providing another option to detect circulating nucleic acids arising from foreign genomes.

In some instances, the foreign molecules are circulating or cell-free nucleic acids (e.g., cell-free DNA, cell-free RNA). In other instances, the subject molecules are circulating or cell-free nucleic acids (e.g., cell-free DNA, cell-free RNA).

As disclosed herein, the molecules may be from circulating foreign cells (e.g., donor cells, bacterial cells, virally infected cells, cancer cells). In some instances, the molecules are from circulating non-foreign cells. In other instances, the molecule is a circulating cell-free molecule. Alternatively, or additionally, the molecules are from an apoptotic cell or a necrotic cell. The molecules may be from a mitotic, post-mitotic, differentiated, redifferentiated, dedifferentiated, stem, pluripotent, or progenitor cell.

In some instances, the foreign molecule is a protein, polypeptide, peptide, or fragment thereof. Additionally, the subject molecule is a protein, polypeptide, peptide, or fragment thereof. Proteins, polypeptides, peptides may comprise cell surface markers (e.g., carbohydrates on bacterial cell walls, receptors), antibodies, transcription factors, translation factors, cell cycle regulators, enzymes, or kinases.

In some instances, the nucleic acid comprises a genetic variation such as a polymorphism. A polymorphism may comprise one or more base changes, an insertion, a repeat, or a deletion of one or more bases. Copy number variants (CNVs), transversions and other rearrangements are also forms of genetic variation. Polymorphic markers include single nucleotide polymorphisms (SNPs), restriction fragment length polymorphisms, variable number of tandem repeats (VNTRs), hypervariable regions, minisatellites, dinucleotide repeats, trinucleotide repeats, tetranucleotide repeats, simple sequence repeats, and insertion elements such as Alu.

The methods disclosed herein often involve conducting a reaction to detect a foreign molecule. In some instances, conducting a reaction to detect a molecule comprises detecting a foreign molecule such as a molecule derived from donor tissue that was transplanted into a subject. Alternatively, conducting a reaction to detect a molecule comprises detecting a molecule that is not a foreign molecule but is derived from a subject, such as a subject who is a transplant recipient. The reaction to detect the molecules may comprise dilution or distribution of a mixture of molecules in the biological sample into discrete sub-samples or individual molecules. For example, the reaction to detect the foreign molecules may comprise a digital PCR reaction. Alternatively, the reaction to detect the molecules does not comprise dilution or distribution of mixture of molecules in the biological sample into discrete sub-samples or individual molecules. For example, the reaction to detect the molecules may comprise direct sequencing of the foreign molecules in the sample comprising a plurality of molecules (e.g., foreign molecules and/or subject molecules).

In some instances, the methods, compositions and systems disclosed herein comprise the isolation of the molecule(s). By way of example only, nucleic acids or proteins are "isolated" when such nucleic acids or proteins are free of at least some of the cellular components with which it is associated in the natural state, or that the nucleic acid or protein has been concentrated to a level greater than the concentration of its in vivo or in vitro production. Nucleic acid can be isolated from the heterogeneous biological sample using techniques well known to those of ordinary skill in the art. See Sambrook, Fritsch and Maniatis, Molecular Cloning: A Laboratory Manual, 2nd edition (1989). In certain embodiments, genomic DNA is isolated from plasma using commercially available kits (e.g., Qiagen Midi Kit or QIAAmp Circulating Nucleic Acid Kit) for purification of DNA from blood cells, following the manufacturer's instructions (QIAmp DNA Blood Midi Kit, Catalog number 51183). DNA is eluted in 1001.11 of distilled water. The Qiagen Midi Kit can also be used to isolate DNA contained in the "buffy coat." In some cases, conducting a reaction can comprise isolation of a foreign nucleic acid from a heterogeneous sample. The isolated foreign nucleic acid can be directly sequenced, thereby digitally separating the foreign genome from the host genome.

In some instances, conducting a reaction to detect a foreign molecule or subject molecule comprises generating a size profile of the molecules, sequencing the molecules, quantifying the molecules, or any combination thereof. For example, methods of the invention comprise the detection of fragments of molecules and conducting a size profile of the molecules. The methods of the invention may also comprise sequencing the foreign molecules. In another example, methods of the invention comprise the use of long-read sequencing technology. In some instances, long-sequencing read technology is used to digitally count whole genomes, or unique regions thereof, contained in a heterogeneous sample.

### Detection Methods

### Sequencing

Many different methods may be used to detect a molecule (e.g., subject molecule or foreign molecule) in a sample. In some instances, the molecules in a heterogeneous sample are detected by sequencing. The methods, compositions, and systems of the invention may comprise sequencing the foreign molecule (e.g., molecules from a pathogen, molecules from a transplanted organ or tissue, molecules from a cancerous cell or tissue, molecules from an unborn fetus). Additionally, subject molecules (e.g., molecules derived from an infected host, molecules derived from a transplant recipient, molecules derived from a non-cancerous cell or tissue, molecules derived from a pregnant female) are sequenced. Any technique for sequencing a nucleic acid known to those skilled in the art can be used in the methods of the provided invention. Sequencing may allow for the presence of multiple genotypes to be detected and quantified in a biological sample containing a mixture of genetic material from different genomic sources. Whole genomes, or unique regions thereof (e.g., genotype patterns such as variable number tandem repeats (VNTRs), short tandem repeats (STRs), and SNP patterns), can be detected and quantified.

In a particular embodiment, the nucleic acid is directly sequenced without diluting the genetic material within the sample or distributing the mixture of genetic material into discrete reaction samples. Sequencing methods may comprise whole genome sequencing or exome sequencing. Sequencing methods such as Maxim-Gilbert, chain-termination, or high-throughput systems may also be used. Additional, suitable sequencing techniques include classic dideoxy sequencing reactions (Sanger method) using labeled terminators or primers and gel separation in slab or capillary, sequencing by synthesis using reversibly terminated labeled nucleotides, pyrosequencing, 454 sequencing, allele specific hybridization to a library of labeled oligonucleotide probes, sequencing by synthesis using allele specific hybridization to a library of labeled clones that is followed by ligation, real time monitoring of the incorporation of labeled nucleotides during a polymerization step, and SOLiD sequencing.

Preferably, the sequencing technique used in the methods of the invention generates at least 100 reads per run, at least 200 reads per run, at least 300 reads per run, at least 400 reads per run, at least 500 reads per run, at least 600 reads per run, at least 700 reads per run, at least 800 reads per run, at least 900 reads per run, at least 1000 reads per run, at least 5,000 reads per run, at least 10,000 reads per run, at least 50,000 reads per run, at least 100,000 reads per run, at least 500,000 reads per run, or at least 1,000,000 reads per run. Alternatively, the sequencing technique used in the methods of the invention generates at least 1,500,000 reads per run, at least 2,000,000 reads per run, at least 2,500,000 reads per run, at least 3,000,000 reads per run, at least 3,500,000 reads per run, at least 4,000,000 reads per run, at least 4,500,000 reads per run, or at least 5,000,000 reads per run.

Preferably, the sequencing technique used in the methods of the invention can generate at least about 30 bp, at least about 40 bp, at least about 50 bp, at least about 60 bp, at least about 70 bp, at least about 80 bp, at least about 90 bp, at least about 100 bp, at least about 110, at least about 120 bp per read, at least about 150 bp, at least about 200 bp, at least about 250 bp, at least about 300 bp, at least about 350 bp, at least about 400 bp, at least about 450 bp, at least about 500 bp, at least about 550 bp, at least about 600 bp, at least about 700 bp, at least about 800 bp, at least about 900 bp, or at least about 1,000 bp per read. Alternatively, the sequencing technique used in the methods of the invention can generate long sequencing reads. In some instances, the sequencing technique used in the methods of the invention can generate at least about 1,200 bp per read, at least about 1,500 bp per read, at least about 1,800 bp per read, at least about 2,000 bp per read, at least about 2,500 bp per read, at least about 3,000 bp per read, at least about 3,500 bp per read, at least about 4,000 bp per read, at least about 4,500 bp per read, at least about 5,000 bp per read, at least about 6,000 bp per read, at least about 7,000 bp per read, at least about 8,000 bp per read, at least about 9,000 bp per read, or at least about 10,000 bp per read.

High-throughput sequencing systems may allow detection of a sequenced nucleotide immediately after or upon its incorporation into a growing strand, i.e., detection of sequence in real time or substantially real time. In some cases, high throughput sequencing generates at least 1,000, at least 5,000, at least 10,000, at least 20,000, at least 30,000, at least 40,000, at least 50,000, at least 100,000 or at least 500,000 sequence reads per hour; with each read being at least 50, at least 60, at least 70, at least 80, at least 90, at least 100, at least 120, at least 150, at least 200, at least 250, at least 300, at least 350, at least 400, at least 450, or at least 500 bases per read. Sequencing can be performed using nucleic acids described herein such as genomic DNA, cDNA derived from RNA transcripts or RNA as a template.

Examples of high throughput sequencing methods include, but are not limited to, Lynx Therapeutics' Massively Parallel Signature Sequencing (MPSS), Polony sequencing, 454 pyrosequencing, Illumina (Solexa) sequencing, SOLiD sequencing, Ion Torrent™, Ion semiconductor sequencing, DNA nanoball sequencing, Helioscope™ single molecule sequencing, Single Molecule SMRT(TM) sequencing, Single Molecule real time (RNAP) sequencing, Nanopore DNA sequencing, and VisiGen Biotechnologies approach.

Suitable sequencing platforms that are useful with methods of the invention include, but are not limited to, True Single Molecule Sequencing (tSMS™) technology such as the HeliScope™ Sequencer offered by Helicos Inc. (Cambridge, MA), Single Molecule Real Time (SMRT™) technology, such as the PacBio RS system offered by Pacific Biosciences (California) and the Solexa Sequencer, Genome Analyzer IIx, HiSeq, and MiSeq offered by Illumina. In the tSMS technique, a DNA sample is cleaved into strands of approximately 100 to 200 nucleotides, and a polyA sequence is added to the 3' end of each DNA strand. Helicos True Single Molecule Sequencing (tSMS) (Harris T. D. et al. (2008) Science 320:106-109). Each strand is labeled by the addition of a fluorescently labeled adenosine nucleotide. The DNA strands are then hybridized to a flow cell, which contains millions of oligo-T capture sites that are immobilized to the flow cell surface. The templates can be at a density of about 100 million templates/cm². The flow cell is then loaded into an instrument, e.g., HeliScope™ sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are detected by imaging the flow cell surface. After imaging, a cleavage step removes the fluorescent label, and the process is repeated with other fluorescently labeled nucleotides until the desired read length is achieved. Sequence information is collected with each nucleotide addition step.

Another example of a sequencing technology that can be used in the methods of the provided invention includes the single molecule, real-time (SMRT™) technology of Pacific Biosciences. In SMRT™, each of the four DNA bases is attached to one of four different fluorescent dyes. These dyes are phospholinked. A single DNA polymerase is immobilized with a single molecule of template single stranded DNA at the bottom of a zero-mode waveguide (ZMW). A ZMW is a confinement structure which enables observation of incorporation of a single nucleotide by DNA polymerase against the background of fluorescent nucleotides that rapidly diffuse in an out of the ZMW (in microseconds). It takes several milliseconds to incorporate a nucleotide into a growing strand. During this time, the fluorescent label is excited and produces a fluorescent signal, and the fluorescent tag is cleaved off. Detection of the corresponding fluorescence of the dye indicates which base was incorporated. The process is repeated.

Another example of a sequencing technology that can be used in the methods of the provided invention is SOLEXA sequencing (Illumina). SOLEXA sequencing is based on the amplification of DNA on a solid surface using fold-back PCR and anchored primers. Genomic DNA is fragmented, and adapters are added to the 5' and 3' ends of the fragments. DNA fragments that are attached to the surface of flow cell channels are extended and bridge amplified. The fragments become double stranded, and the double stranded molecules are denatured. Multiple cycles of the solid-phase amplification followed by denaturation can create several million clusters of approximately 1,000 copies of single-stranded DNA molecules of the same template in each channel of the flow cell. Primers, DNA polymerase and four fluorophore-labeled, reversibly terminating nucleotides are used to perform sequential sequencing. After nucleotide incorporation, a laser is used to excite the fluorophores, and an image is captured and the identity of the first base is recorded. The 3' terminators and fluorophores from each incorporated base are removed and the incorporation, detection and identification steps are repeated.

Another example of a DNA sequencing technique that can be used in the methods of the provided invention is 454 sequencing (Roche) (Margulies, M et al. 2005, Nature, 437, 376-380). 454 sequencing involves two steps. In the first step, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is detected and analyzed.

Another example of a DNA sequencing technique that can be used in the methods of the invention includes the Genome Sequencer FLX systems (Roche/454). The Genome Sequences FLX systems (e.g., GS FLX/FLX+, GS Junior) offer more than 1 million high-quality reads per run and read lengths of 400 bases. These systems are ideally suited for de novo sequencing of whole genomes and transcriptomes of any size, metagenomic characterization of complex samples, or resequencing studies.

SOLiD™ Sequencing is another example of a DNA sequencing technique that can be used in the methods. In SOLiD sequencing, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

In some instances, sequencing comprises paired-end sequencing. Paired-end sequencing can comprise a modification to the standard single-read DNA library preparation, facilitating reading both the forward and reverse template strands of each cluster during one paired-end read. In addition to sequence information, both reads contain long-range positional information, allowing for highly precise alignment of reads and determination of molecule length. The Paired-End Sequencing Assay can utilize a combination of cBot (or the Cluster Station) and the Paired-End Module followed by paired-end sequencing on the Genome Analyzer*_{IIx}* or HiSeq or MiSeq. The paired-end sequencing protocol can also allow the end user to choose the length of the insert (200-500 bp) and sequence either end of the insert, generating highly quality, alignable sequence data. A typical paired-end run can achieve 2 × 150 bp reads and up to 200 million reads.

Nanopore sequencing is another example of a sequencing technique that can be used. A nanopore is a small hole, of the order of 1 nanometer in diameter. Immersion of a nanopore in a conducting fluid and application of a potential across it results in a slight electrical current due to conduction of ions through the nanopore. The amount of current which flows is sensitive to the size of the nanopore. As a DNA molecule passes through a nanopore, each nucleotide on the DNA molecule obstructs the nanopore to a different degree. Thus, the change in the current passing through the nanopore as the DNA molecule passes through the nanopore represents a reading of the DNA sequence.

Another example of a sequencing technique that can be used in the methods of the provided invention involves using a chemical-sensitive field effect transistor (chemFET) array to sequence DNA (for example, as described in US Patent Application Publication No. 20090026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be detected by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

Another example of a sequencing technique that can be used in the methods of the provided invention involves using an electron microscope (Moudrianakis E. N. and Beer M. Proc Natl Acad Sci USA. 1965 March; 53:564-71). In one example of the technique, individual DNA molecules are labeled using metallic labels that are distinguishable using an electron microscope. These molecules are then stretched on a flat surface and imaged using an electron microscope to measure sequences.

In some instances, high-throughput sequencing involves the use of technology available by Helicos Biosciences Corporation (Cambridge, Massachusetts) such as the Single Molecule Sequencing by Synthesis (SMSS) method. SMSS is unique because it allows for sequencing the entire human genome with no pre-amplification step needed. Thus, distortion and nonlinearity in the measurement of nucleic acids are reduced. Sequencing methods may also allow for detection of a SNP nucleotide in a sequence in substantially real time or real time.

Alternatively, high-throughput sequencing involves the use of technology available by 454 Lifesciences, Inc. (Branford, Connecticut) such as the Pico Titer Plate device which includes a fiber optic plate that transmits chemiluminescent signal generated by the sequencing reaction to be recorded by a CCD camera in the instrument. This use of fiber optics allows for the detection of a minimum of 20 million base pairs in 4.5 hours.

High-throughput sequencing may be performed using Clonal Single Molecule Array (Solexa, Inc.) or sequencing-by-synthesis (SBS) utilizing reversible terminator chemistry. High-throughput sequencing of RNA or DNA can take place using AnyDot.chips (Genovoxx, Germany), which allows for the monitoring of biological processes (e.g., miRNA expression or allele variability (SNP detection). In particular, the AnyDot-chips allow for 10x - 50x enhancement of nucleotide fluorescence signal detection.

Other high-throughput sequencing systems include those disclosed in Venter, J., et al. Science 16 February 2001; Adams, M. et al, Science 24 March 2000; and M. J, Levene, et al. Science 299:682-686, January 2003; as well as US Publication Application No. 20030044781 and 2006/0078937. In general, high-throughput sequencing systems involve sequencing a target nucleic acid molecule having a plurality of bases by the temporal addition of bases via a polymerization reaction that is measured on a molecule of nucleic acid, e.g., the activity of a nucleic acid polymerizing enzyme on the template nucleic acid molecule to be sequenced is followed in real time. Sequence can then be deduced by identifying which base is being incorporated into the growing complementary strand of the target nucleic acid by the catalytic activity of the nucleic acid polymerizing enzyme at each step in the sequence of base additions. A polymerase on the target nucleic acid molecule complex is provided in a position suitable lo move along the target nucleic acid molecule and extend the oligonucleotide primer at an active site. A plurality of labeled types of nucleotide analogs are provided proximate to the active site, with each distinguishably type of nucleotide analog being complementary to a different nucleotide in the target nucleic acid sequence. The growing nucleic acid strand is extended by using the polymerase to add a nucleotide analog to the nucleic acid strand at the active site, where the nucleotide analog being added is complementary to the nucleotide of the target nucleic acid at the active site. The nucleotide analog added to the oligonucleotide primer as a result of the polymerizing step is identified. The steps of providing labeled nucleotide analogs, polymerizing the growing nucleic acid strand, and identifying the added nucleotide analog are repeated so that the nucleic acid strand is further extended and the sequence of the target nucleic acid is determined.

Shotgun sequencing may be performed to detect molecules in a heterogeneous sample. In shotgun sequencing, DNA is broken up randomly into numerous small segments, which are sequenced using the chain termination method to obtain reads. Multiple overlapping reads for the target DNA are obtained by performing several rounds of this fragmentation and sequencing. Computer programs then use the overlapping ends of different reads to assemble them into a continuous sequence

Sequencing techniques may also be used for detection and quantitation of SNPs. In this case, one can estimate the sensitivity of detection. There are two components to sensitivity: (i) the number of molecules analyzed (depth of sequencing) and (ii) the error rate of the sequencing process. Regarding the depth of sequencing, a frequent estimate for the variation between individuals is that about one base per thousand differs. Currently, sequencers such as the Illumina Genome Analyzer have read lengths exceeding 36 base pairs. Without intending to be limited to any theory or specific embodiment, this means that roughly one in 30 molecules analyzed will have a potential SNP. While the fraction of foreign nucleic acid molecules in a heterogeneous sample from a subject is currently not well determined and will depend on organ type, one can take 1% as a baseline estimate based on the literature and applicants own studies with heart transplant patients. At this fraction of foreign nucleic acid molecules, approximately one in 3,000 molecules analyzed will be from the foreign subject (e.g., donor, pathogen, cancer) and informative about donor genotype. On the Genome Analyzer one can obtain about 10 million molecules per analysis channel and there are 8 analysis channels per instrument run. Therefore, if one sample is loaded per channel, one should be able to detect about 3,000 molecules that can be identified as from the donor in origin, more than enough to make a precise determination of the fraction of donor DNA using the above parameters. If one wants to establish a lower limit of sensitivity for this method by requiring at least 100 donor molecules to be detected, then it should have a sensitivity capable of detecting donor molecules when the donor fraction is as low as 0.03%. Higher sensitivity can be achieved simply by sequencing more molecules, i.e. using more channels.

The sequencing error rate also affects the sensitivity of this technique. For an average error rate of ε, the chance of a single SNP being accidentally identified as of donor origin as a result of a misread is roughly ε/3. For each individual read, this establishes a lower limit of sensitivity of one's ability to determine whether the read is due to donor or recipient. Typical sequencing error rates for base substitutions vary between platforms, but are between 0.5-1.5%. This places a potential limit on sensitivity of 0.16 to 0.50%. However, it is possible to systematically lower the sequencing error rate by resequencing the sample template multiple times, as has been demonstrated by Helicos Biosciences (Harris, T.D., et al., Science, 320, 106-109 (2008)). A single application of resequencing would reduce the expected error rate of donor SNP detection to ε²/9 or less than .003%.

### Genotyping

In some embodiments, the methods of the invention are used to genotype the donor and/or the recipient before transplantation to enable the detection of donor-specific nucleic acids such as DNA or RNA in bodily fluids such as blood or urine from the organ recipient after transplantation. Sequencing performed on the nucleic acid recovered from plasma or other biological samples may directly quantitate the percentage of donor-specific species within the sample. This approach allows for a reliable identification of sequences arising solely from the organ transplantation that can be made in a manner that is independent of the genders of donor and recipient.

The sequencing methods described herein are useful for generating a genetic fingerprint for the donor organ, tissue, or cell and/or a genetic fingerprint for the transplant recipient. Genotyping of transplant donors and transplant recipients prior to transplantation establishes a profile, using distinguishable markers, for detecting donor nucleic acids (e.g. circulating cell-free nucleic acid or nucleic acids from circulating donor cells). In some embodiments, for xenotransplants, nucleic acids from the donors can be mapped to the genome of the donor species.

Following transplantation, samples as described herein can be drawn from the patient and analyzed for the donor genetic fingerprint and/or the transplant recipient genetic fingerprint. The proportion of donor nucleic acids can be monitored over time and an increase in this proportion can be used to determine transplant status or outcome (e.g. transplant rejection).

In some embodiments, genotyping comprises whole genome sequencing and quantitation of nucleic acids from circulating transplant donor cells or circulating cell-free nucleic acids._In some embodiments, genotyping comprises detection and quantitation of polymorphic markers. Examples of polymorphic markers include single nucleotide polymorphisms (SNPs), restriction fragment length polymorphisms (RFLPs), variable number of tandem repeats (VNTRs), short tandem repeats (STRs), hypervariable regions, minisatellites, dinucleotide repeats, trinucleotide repeats, tetranucleotide repeats, simple sequence repeats, and insertion elements such as Alu. In some embodiments, genotyping comprises detection and quantitation of STRs. In some embodiments, genotyping comprises detection and quantitation of VNTRs.

In some instances, genotyping comprises genotyping foreign molecules. In some instances, genotyping comprises genotyping non-foreign molecules. In some instances, non-foreign molecules are genotyped and homozygous non-foreign positions are monitored for foreign bases. In some instances, the foreign molecules are not genotyped. In some embodiments, genotyping comprises detection and quantitation of SNPs. Without intending to be limited to any theory, any donor and recipient will vary at roughly three million SNP positions if fully genotyped. Usable SNPs must be homozygous for the recipient and ideally homozygous for the donor as well. While the majority of these positions will contain SNPs that are heterozygous for either the donor or the recipient, over 10% (or hundreds of thousands) will be homozygous for both donor and recipient meaning a direct read of that SNP position can distinguish donor DNA from recipient DNA. For example, after genotyping a transplant donor and transplant recipient, using existing genotyping platforms know in the art including the one described herein, one could identify approximately 1.2 million total variations between a transplant donor and transplant recipient. Usable SNPs may comprise approximately 500,000 heterozygous donor SNPs and approximately 160,000 homozygous donor SNPs.

Due to the low number of expected reads for any individual nucleic acid (e.g. SNP) in patient samples, some preamplification of the sample material may be required before analysis to increase signal levels, but using either preamplification, sampling more target nucleic acid positions (e.g. SNP positions), or both, will provide a reliable read-out of the transplant donor nucleic acid fraction. Preamplification can be performed using any suitable method known in the art such as multiple displacement amplification (MDA) (Gonzalez et al. Environ Microbiol; 7(7); 1024-8 (2005)) or amplification with outer primers in a nested PCR approach. This permits detection and analysis of donor nucleic acids even if the total amount of donor nucleic acid in the sample (e.g. blood from transplant patient) is only up to 1000 ng, 500 ng, 200 ng, 100 ng, 50 ng, 40 ng, 30 ng, 20 ng, 10 ng, 5 ng, 1 ng, 500 pg, 200 pg, 100 pg, 50 pg, 40 pg, 30 pg, 20 p, 10 pg, 5 pg, or 1 pg or between 1-5 µg, 5-10 µg, or 10-50 µg.

Methods, compositions and systems disclosed herein can provide for digital counting of whole genomes, or unique regions thereof. Methods, compositions and systems disclosed herein can afford more data via long sequence reads than traditional methods of analysis, such as PCR, SNP arrays, restriction fragment length polymorphism identification (RFLPI) of genomic DNA, random amplified polymorphic detection (RAPD) of genomic DNA, and amplified fragment length polymorphism detection (AFLPD). As such, methods of the invention can deliver a higher detection rate of circulating nucleic acids in a host subject and improved clinical performance compared to conventional screening methods.

### Amplification

In some instances, the methods disclosed herein comprise amplification of the foreign molecules. Additionally, or alternatively, the methods disclosed herein comprise amplification of the subject-derived molecules. The RNA or DNA molecules may be cell-free. Alternatively, the RNA or DNA molecules are isolated from a cell. Amplification methods disclosed herein may be combined with reverse transcription to quantify and detect a foreign and/or subject-derived RNA molecule. In some instances, amplification comprises a PCR-based method. In some instances, the PCR-based method is PCR, quantitative PCR, emulsion PCR, droplet PCR, hot start PCR, in situ PCR, inverse PCR, multiplex PCR, Variable Number of Tandem Repeats (VNTR) PCR, asymmetric PCR, long PCR, nested PCR, hemi-nested PCR, touchdown PCR, assembly PCR, or colony PCR.

In other instances, amplification comprises a non-PCR-based method. In some instances, the non-PCR-based method is multiple displacement amplification (MDA), transcription-mediated amplification (TMA), nucleic acid sequence-based amplification (NASBA), strand displacement amplification (SDA), real-time SDA, rolling circle amplification, or circle-to-circle amplification.

### Quantitative methods

In some instances, the methods, compositions and systems disclosed herein comprise the use of quantitative methods for the detection of the molecules (e.g., DNA, RNA) in the sample. In some instances, the methods, compositions and systems disclosed herein comprise the use of quantitative methods for the detection of foreign molecules. Alternatively, or additionally, the methods, compositions, and systems disclosed herein comprise the use of quantitative methods for the detection of subject-derived molecules. Foreign molecules and subject-derived molecules may be RNA or DNA molecules. The RNA or DNA molecules may be cell-free. Alternatively, the RNA or DNA molecules are isolated from a cell. Examples of quantitative methods include, but are not limited to, qPCR, digital PCR, nanoreporters, and chromatography. Quantitative methods can be used to determine the percentage of molecules in a heterogeneous sample. The relative or absolute amounts of the molecules may also be determined by the quantitative methods disclosed herein.

Quantitative PCR (e.g., qPCR, RTQ-PCR) may be used to determine the amount of the foreign molecules in a heterogeneous sample. Alternatively, or additionally, quantitative PCR (e.g., qPCR, RTQ-PCR) may be used to determine the amount of subject-derived molecules in a heterogeneous sample. Generally, qPCR is used to amplify and simultaneously quantify a DNA molecule. In some instances, qPCR may be combined with reverse transcription to quantify and detect an RNA molecule. qPCR follows the same general principle of polymerase chain reaction, however, qPCR allows real time detection of the amplified molecule (e.g., as the reaction progresses, the amplified product may be detected). Two common methods for detection of products in qPCR are: (1) non-specific fluorescent dyes that intercalate with any double-stranded DNA, and (2) sequence-specific DNA probes consisting of oligonucleotides that are labeled with a fluorescent reporter which permits detection only after hybridization of the probe with its complementary DNA target. qPCR can be used to quantify nucleic acids by two methods: relative quantification and absolute quantification. However, both relative and absolute quantification are comparative methods. Relative quantification is based on internal reference genes to determine fold-differences in expression of the target gene. Absolute quantification gives the exact number of target DNA molecules by comparison with DNA standards.

Digital PCR and/or next-generation sequencing methods allows for the presence of multiple genotypes to be detected and quantified in a biological sample containing a mixture of genetic material from different genomic sources. Partial or whole genomes, or unique regions thereof (e.g., genotype patterns such as variable number tandem repeats (VNTRs), short tandem repeats (STRs), and SNP patterns), can be detected and quantified.

Digital PCR (dPCR) may be used to directly quantify and clonally amplify nucleic acids including DNA, cDNA or RNA. dPCR also carries out a single reaction within a sample, however the sample is separated into a large number of partitions and the reaction is carried out in each partition individually. The partitioning of the sample allows one to count the molecules by estimating according to Poisson. As a result, each part will contain "0" or "1" molecules, or a negative or positive reaction, respectively. After PCR amplification, nucleic acids may be quantified by counting the regions that contain PCR end-product, positive reactions. In conventional PCR, starting copy number is proportional to the number of PCR amplification cycles. dPCR, however, is not dependent on the number of amplification cycles to determine the initial sample amount, eliminating the reliance on uncertain exponential data to quantify target nucleic acids and providing absolute quantification. Unlike qPCR, absolute quantification by dPCR is not a comparative method.

In some instances, quantitation of molecules comprises absolute quantitation. Absolute quantitation may comprise use of one or more control oligonucleotide species. The one or more control oligonucleotide species can be added to a sample from the subject. The one or more control oligonucleotide species can be of comparable size to the molecules expected in the cell-free molecules (e.g., DNA, RNA) samples. The one or more control oligonucleotide species can have distinct sequence identity. The one or more control oligonucleotide species can have non-natural sequence identity. The one or more control oligonucleotide species may comprise at least one deoxyribonucleotide and/or ribonucleotide. The one or more control oligonucleotide species can have one or more non-natural nucleotides. The samples with the control oligonucleotide species can be prepared and sequenced by any of the methods disclosed herein. The percentage of foreign molecules and the percentage of control oligonucleotide species can be calculated. The number of observed control oligonucleotide species can be used to calculate the number of molecules in the starting sample. Consequently, the absolute number of foreign molecules in the starting sample can be calculated. Control oligonucleotide species ratios and/or control oligonucleotide species lengths can be used to account for the biases resulting from different sample inputs. Alternatively, or additionally, the percentage of subject-derived molecules in the starting sample can be calculated.

Another method to quantify molecules may comprise the use of unique identifiers (e.g., barcodes, nanoreporters, labels). Generally, the molecules are labeled with unique identifiers and the uniquely labeled molecules may be detected by methods such as sequencing, ELISAs, and arrays and quantified. In some instances, the unique identifiers are nanoreporters, such as those described in US patent number 7,473,767, US publication number 2007/0166708, US application number 11/645,270, and PCT application number US06/049274. The unique identifiers may comprise nucleic acids, biomolecules, peptides, enzymes, kinases, proteins, antibodies, or antigens. The unique identifiers may be attached to the molecules and attachment may occur by ligation, binding, covalent attachment, hybridization or PCR. For example, a unique identifier comprising a nucleic acid may be ligated to a molecule (e.g., nucleic acid). In another example, a unique identifier comprising an antibody may be bound to a molecule (e.g., protein). Alternatively, the unique identifier is hybridized to the molecule.

In some instances, the molecule may be fragmented. Fragmentation of the molecules can occur by sonication, needle shear, nebulisation, shearing (e.g., acoustic shearing, mechanical shearing, point-sink shearing), passage through a French pressure cell, or enzymatic digestion. Enzymatic digestion may occur by nuclease digestion (e.g., micrococcal nuclease digestion, endonucleases, exonucleases, RNAse H or DNase I). Fragmentation of the target nucleic acid may result in fragment sized of about 100 bp to about 2000 bp, about 200 bp to about 1500 bp, about 200 bp to about 1000 bp, about 200 bp to about 500 bp, about 500 bp to about 1500 bp, and about 500 bp to about 1000 bp.

The detection, identification and/or quantitation of the molecules can be performed using arrays (e.g. SNP arrays). Results can be visualized using a scanner that enables the viewing of intensity of data collected and software to detect and quantify nucleic acid. Such methods are disclosed in part US Patent No. 6,505,125. Another method contemplated by the present invention to detect and quantify nucleic acids involves the use of beads as is commercially available by Illumina, Inc. (San Diego) and as described in US Patent Nos. 7,035,740; 7033,754; 7,025,935, 6,998,274; 6,942,968; 6,913,884; 6,890,764; 6,890,741; 6,858,394; 6,812,005; 6,770,441; 6,620,584; 6,544,732; 6,429,027; 6,396,995; 6,355,431 and US Publication Application Nos. 20060019258; 20050266432; 20050244870; 20050216207;20050181394;20050164246;20040224353;20040185482;20030198573;20030175773; 20030003490; 20020187515; and 20020177141; and in B. E. Stranger, et al., Public Library of Science-Genetics, I (6), December 2005; Jingli Cai, et al., Stem Cells, published online November 17, 2005; C.M. Schwartz, et al., Stem Cells and Development, f4, 517-534, 2005; Barnes, M., J. et al., Nucleic Acids Research, 33 (1 81, 5914-5923, October 2005; and Bibikova M, et al. Clinical Chemistry, Volume 50, No. 112, 2384-2386, December 2004. Additional description for preparing RNA for bead arrays is described in Kacharmina JE, et al., Methods Enzymol. 303: 3-18, 1999; Pabon C, et al., Biotechniques 3 1(4): 8769,2001; Van Gelder RN, et al., Proc Natl Acad Sci USA 87: 1663-7 (1990); and Murray, SS. BMC Genetics B(SupplI):SX5 (2005).

When analyzing SNPs according to the methods described herein, the foreign and/or subject nucleic acids can be labeled and hybridized with a DNA microarray (e.g., 100K Set Array or other array). Results can be visualized using a scanner that enables the viewing of intensity of data collected and software "calls" the SNP present at each of the positions analyzed. Computer implemented methods for determining genotype using data mapping arrays are disclosed, for example, in Liu, et al., Bioinformatics 19:2397-2403,2003; and Di et al., Bioinformatics 21 : 1958-63,2005. Computer implemented methods for linkage analysis using mapping array data are disclosed, for example, in Ruschendorf and Nusnberg, Bioinformatics 21:2123-5, 2005; and Leykin et al., BMC Genet. 6:7, 2005; and in US Patent No. 5,733,729.

In some instances, genotyping microarrays that are used to detect SNPs can be used in combination with molecular inversion probes (MIPs) as described in Hardenbol et al., Genome Res. 15(2) :269-275,2005; Hardenbol, P. et al. Nature Biotechnology 21(6), 673-8,2003; Faham M, et al. Hum Mol Genet. 10(16):1657-64,2001; Maneesh Jain, Ph.D., et al. Genetic Engineering News V24: No. 18, 2004; Fakhrai-Rad H, et al. Genome Res. Jul; 14(7): 1404-12, 2004; and in U.S. Pat. No. 5,858,412. Universal tag arrays and reagent kits for performing such locus specific genotyping using panels of custom MIPs are available from Affymetrix and Par Allele. MIP technology involves the use of enzymological reactions that can score up to 10,000; 20,000; 50,000; 100,000; 200,000; 500,000; 1,000,000; 2,000,000 or 5,000,000 SNPs (target nucleic acids) in a single assay. The enzymological reactions are insensitive to cross-reactivity among multiple probe molecules and there is no need for pre-amplification prior to hybridization of the probe with the genomic DNA. In some instances, the molecules or SNPs can be obtained from a single cell.

Electrophoretic methods may also be used to detect and/or quantify molecules. In some instances, the methods, compositions and systems disclosed herein comprise electrophoretic detection and/or quantitation of foreign molecules. Alternatively, or additionally, the methods, compositions and systems disclosed herein comprise electrophoretic detection and/or quantitation of subject-derived molecules. For example, gel electrophoresis is used to detect nucleic acids and proteins and includes overlay gel electrophoresis, charge shift method, band shift assay, countermigration electrophoresis, affinophoresis, affinity electrophoresis, rocket immunoelectrophoresis, and crossed immunoelectrophoresis. Another example of an electrophoretic method is capillary electrophoresis. Affinity capillary electrophoresis has demonstrated its value in the measurement of binding constants, the estimation of kinetic rate constants, and the determination of stoichiometry of biomolecular interactions. It offers short analysis time, requires minute amounts of protein samples, usually involves no radiolabeled compounds, and, most importantly, is carried out in solution. SDS-PAGE may also be used to detect nucleic acid molecules. Electrophoretic methods may also be used to generate a size profile of the molecules, thereby detecting foreign molecules.

Additional methods for quantifying molecules include, but are not limited to, gas chromatography, supercritical fluid chromatography, liquid chromatography (including partition chromatography, adsorption chromatography, ion exchange chromatography, size exclusion chromatography, thin-layer chromatography, and affinity chromatography), electrophoresis (including capillary electrophoresis, capillary zone electrophoresis, capillary isoelectric focusing, capillary electrochromatography, micellar electrokinetic capillary chromatography, isotachophoresis, transient isotachophoresis and capillary gel electrophoresis), comparative genomic hybridization (CGH), microarrays, bead arrays, and high-throughput genotyping such as with the use of molecular inversion probe (MIP). Southern blot and Northern blot may also be used to detect and/or quantify nucleic acid molecules. ELISA, immunofluorescence, and Western blot are additional methods that may be used to detect and/or quantify biomolecules (e.g., proteins).

The quantification method may involve amplification; although, in some cases, the method may be amplification independent. Cylindrical illumination confocal spectroscopy and molecular barcoding are examples of quantification methods that can be conducted in an amplification-independent manner.

Alternatively, fluorescent dyes may also be used for the detection and/or quantification of molecules. Fluorescent dyes may typically be divided into families, such as fluorescein and its derivatives; rhodamine and its derivatives; cyanine and its derivatives; coumarin and its derivatives; Cascade Blue™ and its derivatives; Lucifer Yellow and its derivatives; BODIPY and its derivatives; and the like. Exemplary fluorophores include indocarbocyanine (C3), indodicarbocyanine (C5), Cy3, Cy3.5, Cy5, Cy5.5, Cy7, Texas Red, Pacific Blue, Oregon Green 488, Alexa Fluor®-355, Alexa Fluor 488, Alexa Fluor 532, Alexa Fluor 546, Alexa Fluor-555, Alexa Fluor 568, Alexa Fluor 594, Alexa Fluor 647, Alexa Fluor 660, Alexa Fluor 680, JOE, Lissamine, Rhodamine Green, BODIPY, fluorescein isothiocyanate (FITC), carboxy-fluorescein (FAM), phycoerythrin, rhodamine, dichlororhodamine (dRhodamine), carboxy tetramethylrhodamine (TAMRA), carboxy-X-rhodamine (ROX.TM.), LIZ™, VIC™., NED™, PET™, SYBR, PicoGreen, RiboGreen, and the like. Descriptions of fluorophores and their use, can be found in, among other places, R. Haugland, Handbook of Fluorescent Probes and Research Products, 9.sup.th ed. (2002), Molecular Probes, Eugene, Oreg.; M. Schena, Microarray Analysis (2003), John Wiley & Sons, Hoboken, N.J.; Synthetic Medicinal Chemistry 2003/2004 Catalog, Berry and Associates, Ann Arbor, Mich.; G. Hermanson, Bioconjugate Techniques, Academic Press (1996); and Glen Research 2002 Catalog, Sterling, Va. Near-infrared dyes are expressly within the intended meaning of the terms fluorophore and fluorescent reporter group.

In another aspect of the invention, a branched-DNA (bDNA) approach is used to increase the detection sensitivity. In some instances, bDNA approach is applied to an array detection assay. The array detection assay can be any array assay known in the art, including the array assays described herein. bDNA approach amplifies the signals through a branched DNA that are attached by tens or hundreds of alkaline phosphatase molecules. Thus, the signals are significantly amplified while the fidelity of the original nucleic acid target abundance is maintained.

### Marker profile

In some instances, detection of the molecules may comprise producing a marker profile. The marker profile may comprise one or more molecules or fragments thereof. In some instances, producing a marker profile comprises sequencing the foreign molecules. The foreign molecules may be DNA or RNA molecules. The DNA or RNA molecules may be from a cell. Alternatively, the DNA or RNA molecules are cell-free molecules. The marker profile may comprise at least about 1, at least about 2, at least about 3, at least about 4, at least about 5, at least about 10, at least about 15, at least about 20, at least about 30, at least about 40, at least about 50, at least about 100, at least about 200, at least about 300, at least about 400, at least about 500, at least about 600, at least about 700, at least about 800, at least about 900, or at least about 1000 foreign molecules. The foreign molecules may be identical, similar, or different. Identical foreign molecules may comprise the same sequence (nucleotide or peptide sequence). In some instances, the sequence of two or more foreign molecules in the marker profile are at least about 10%, 20%, 30%, 40%, 50%, 60%, 70%, 75%, 80%, 90%, 95%, or 97% identical. The sequence of two or more foreign may overlap (partially or fully). The sequence of two or more foreign molecules may be different. In some instances, the sequence of two or more foreign molecules may be less than about 1%, 5%, 10%, 15%, 20%, 25%, 30%, 35%, 40%, 50%, 60%, 70%, 75%, 80%, 90%, 95%, or 97% identical.

Alternatively, or additionally, producing a marker profile comprises sequencing the subject molecules. The subject molecules may be DNA or RNA molecules. The DNA or RNA molecules may be from a cell. Alternatively, the DNA or RNA molecules are cell-free molecules. The marker profile may comprise at least about 1, at least about 2, at least about 3, at least about 4, at least about 5, at least about 10, at least about 15, at least about 20, at least about 30, at least about 40, at least about 50, at least about 100, at least about 200, at least about 300, at least about 400, at least about 500, at least about 600, at least about 700, at least about 800, at least about 900, or at least about 1000 subject molecules.

The methods disclosed herein may comprise the detection of at least about 10%, 20%, 30%, 40%, 50%, 60%, 70%, 80%, 90%, 95%, or 97% of the foreign molecules in the heterogeneous sample. In some instances, the foreign molecules comprise less than about 90%, 80%, 70%, 60%, 50%, 40%, 30%, or 20% of the total molecules in the sample. Preferably, the foreign molecules comprise less than about 15%, 10%, 9%, 8%, 7%, 6%, 5%, 4%, 3%, 2%, or 1% of the total molecules in the sample. In some instances, the foreign molecules comprise less than about 1%, 0.5%, 0.25%, 0.1% of the total molecules in the sample.

The methods disclosed herein may comprise the detection of at least about 10%, 20%, 30%, 40%, 50%, 60%, 70%, 80%, 90%, 95%, or 97% of the subject molecules in the heterogeneous sample. In some instances, the subject molecules comprise less than about 90%, 80%, 70%, 60%, 50%, 40%, 30%, or 20% of the total molecules in the sample. Alternatively, the subject molecules may comprise less than about 15%, 10%, 9%, 8%, 7%, 6%, 5%, 4%, 3%, 2%, or 1% of the total molecules in the sample. In some instances, the subject molecules comprise greater than about 95%, 96%, 97%, 98%, 98.5%, 99%, 99.5%, or 99.9% of the total molecules in the sample.

### Size Profile

In some instances, the methods disclosed herein comprise generating a size profile of the molecules. Generating a size profile may comprise electrophoretic separation of the molecules and/or sequencing the molecules. The size profile may comprise nucleic acid fragments (e.g., DNA fragments, RNA fragments). In some instances, the nucleic acid fragments comprise DNA fragments. Alternatively, the nucleic acid fragments comprise RNA fragments. In some instances, the nucleic acid fragments are cell-free nucleic acid fragments. Alternatively, the nucleic acid fragments are from a cell. The nucleic acid fragments may be foreign molecules. The nucleic acid molecules may be subject-derived molecules. The nucleic acid fragments may form a ladder of sizes. In some instances, the ladder of sizes comprises nucleic acid fragments of about 180bp increments. For example, the ladder may comprise of nucleic acid fragments of about 180bp, about 360bp, about 540bp, about 720bp, about 900bp, about 1080bp, about 1260bp, about 1440bp, about 1620bp, about 1800bp, etc.

In some instances, for a urine sample, a different use of sizing may involve filtering DNA from the urinary system from DNA coming from other parts of the body (e.g., an organ such as a heart, lung, or liver). Healthy kidneys can normally function to filter out DNA from the blood, though, in some instances, small DNA fragments pass through the kidney. Donor-derived signal can be enriched by isolating sequences, either experimentally or informatically, that are less than about 25, 50, 75, 100, 125, or 150 base pairs. Similarly, kidney-derived or other urinary tract DNA could be enriched by selecting molecules that are at least about 150, 200, 300, or 500 base pairs in length. In some embodiments, the use of both small and/or large DNA fragments may be used to inform patient treatment. For example, the amount of donor DNA observed in small fragments reveals that the donor graft is healthy, thereby resulting in a reduction in an immunosuppressive therapy. In another example, an increase in kidney-derived DNA is indicative of drug nephrotoxicity, thereby resulting in a reduction in an immunosuppressive therapy and/or administration of a new immunosuppressive therapy.

In some instances, the size distribution of the foreign molecules is assayed by sequencing. In addition, the subject-derived molecules are also assayed by sequencing. Sequencing, such as paired-end sequencing, can be performed on the molecules. Identification of subject and foreign-specific SNPs can be used to identify all foreign-derived molecules. The size of the foreign molecules can be calculated from the paired-end sequence alignment. A histogram of sizes can be made. In some instances, features of the size distribution, including mean/median size and the extent of apoptotic ladder-like patterning, is used to determine the ratio of apoptotic versus necrotic contribution. The size distribution and/or the ratio of apoptotic versus necrotic contribution can be used to perform differential diagnosis of different causes of graft injury. Optionally, overall foreign molecule levels, sequences from an infectious agent, and/or sequences from an immune repertoire are also used to perform differential diagnosis of different causes of graft injury.

In some instances, the size profile comprises at least about 1, at least about 2, at least about 3, at least about 4, at least about 5, at least about 10, at least about 15, at least about 20, at least about 25, at least about 30, at least about 35, at least about 40, at least about 45, at least about 50, at least about 60, at least about 70, at least about 80, at least about 90, or at least about 100 molecules. Alternatively, the size profile comprises at least about 500, at least about 1,000, at least about 2,000, at least about 3,000, at least about 4,000, at least about 5,000, at least about 6000, at least about 7,000, at least about 8,000, at least about 9,000, at least about 10,000, at least about 15,000, at least about 20,000, at least about 25,000, at least about 30,000, at least about 40,000, at least about 50,000, at least about 75,000, or at least about 100,000 molecules. Additional information about size profiles can be found in other sections of the present disclosure, such as sections relating to organ transplantation. Such size profiles can also be used for diseases or conditions outside of the organ transplantation setting.

### Analysis

After digitally counting the number of genomes and/or genotype patterns present in the heterogeneous biological sample, ratios of the different genomes and/or genotype patterns can then be compared to determine the relative amounts of the various genotypes and/or genotype patterns in the biological sample. By counting the number of genomes and/or genotype patterns, the over- or underrepresentation of any foreign genome within a given individual can be detected. It should be noted that methods of the invention do not require the differentiation of foreign versus host nucleic acid, and with large enough sequence counts, methods of the invention can be applied to arbitrarily small fractions of foreign nucleic acid.

High-throughput analysis can be achieved using one or more bioinformatics tools, such as ALLPATHS (a whole genome shotgun assembler that can generate high quality assemblies from short reads), Arachne (a tool for assembling genome sequences from whole genome shotgun reads, mostly in forward and reverse pairs obtained by sequencing cloned ends, BACCardl (a graphical tool for the validation of genomic assemblies, assisting genome finishing and intergenome comparison), CCRaVAT & QuTie (enables analysis of rare variants in large-scale case control and quantitative trait association studies), CNV-seq (a method to detect copy number variation using high throughput sequencing), Elvira (a set of tools/procedures for high throughput assembly of small genomes (e.g., viruses)), Glimmer (a system for finding genes in microbial DNA, especially the genomes of bacteria, archaea and viruses), gnumap (a program designed to accurately map sequence data obtained from nextgeneration sequencing machines), Goseq (an R library for performing Gene Ontology and other category based tests on RNA-seq data which corrects for selection bias), ICAtools (a set of programs useful for medium to large scale sequencing projects), LOCAS (a program for assembling short reads of second generation sequencing technology), Maq (builds assembly by mapping short reads to reference sequences, MEME (motif-based sequence analysis tools, NGSView (allows for visualization and manipulation of millions of sequences simultaneously on a desktop computer, through a graphical interface, OSLay (Optimal Syntenic Layout of Unfinished Assemblies), Perm (efficient mapping for short sequencing reads with periodic full sensitive spaced seeds, Projector (automatic contig mapping for gap closure purposes), Qpalma (an alignment tool targeted to align spliced reads produced by sequencing platforms such as Illumina, Solexa, or 454), RazerS (fast read mapping with sensitivity control), SHARCGS (SHort read Assembler based on Robust Contig extension for Genome Sequencing; a DNA assembly program designed for *de novo* assembly of 25-40mer input fragments and deep sequence coverage), Tablet (next generation sequence assembly visualization), and Velvet (sequence assembler for very short reads).

The methods described herein are used to detect and/or quantify whole genomes or genomic DNA regions. In some embodiments, the methods described herein can discriminate and quantitate genomic DNA regions. The methods described herein can discriminate and quantitate at least 1; 2; 3; 4; 5; 10, 20; 50; 100; 200; 500; 1,000; 2,000; 5,000; 10,000, 20,000; 50,000; 100,000; 200,000; 300,000; 400,000; 500,000; 600,000; 700,000; 800,000; 900,000; 1,000,000; 2,000,000 or 3,000,000 different genomic DNA regions. The methods described herein can discriminate and quantitate genomic DNA regions varying by 1 nt or more than 1, 2, 3, 5, 10, 15, 20, 21, 22, 24, 25, 30, 40, 50, 60, 70, 80, 90, 100, 200, 300, 400 or 500 nt.

In some cases, the methods described herein are used to detect and/or quantify genomic DNA regions such as a region containing a DNA polymorphism. A polymorphism refers to the occurrence of two or more genetically determined alternative sequences or alleles in a population. A polymorphic marker or site is the locus at which divergence occurs. Preferred markers have at least two alleles, each occurring at a frequency of preferably greater than 1%, and more preferably greater than 10% or 20% of a selected population. A polymorphism may comprise one or more base changes, an insertion, a repeat, or a deletion. A polymorphic locus may be as small as one base pair. Polymorphic markers include single nucleotide polymorphisms (SNPs), restriction fragment length polymorphisms (RFLPs), short tandem repeats (STRs), variable number of tandem repeats (VNTRs), hypervariable regions, minisatellites, dinucleotide repeats, trinucleotide repeats, tetranucleotide repeats, simple sequence repeats, and insertion elements such as Alu. A polymorphism between two nucleic acids can occur naturally, or be caused by exposure to or contact with chemicals, enzymes, or other agents, or exposure to agents that cause damage to nucleic acids, for example, ultraviolet radiation, mutagens or carcinogens.

In some cases, the methods described herein can discriminate and quantitate a DNA region containing a DNA polymorphism. The methods described herein can discriminate and quantitate of at least 1; 2; 3; 4; 5; 10, 20; 50; 100; 200; 500; 1,000; 2,000; 5,000; 10,000, 20,000; 50,000; 100,000; 200,000; 300,000; 400,000; 500,000; 600,000; 700,000; 800,000; 900,000; 1,000,000; 2,000,000 or 3,000,000 DNA polymorphism. In some embodiments, the methods described herein can discriminate and quantitate at least 1; 2; 3; 4; 5; 10; 20; 50; 100; 200; 500; 1,000; 2,000; 5,000; 10,000; 20,000; 50,000; 100,000; 200,000; 300,000; 400,000; 500,000; 600,000; 700,000; 800,000; 900,000; 1,000,000; 2,000,000 or 3,000,000 different polymorphic markers._In some embodiments, the methods described herein can discriminate and quantitate at least 1; 2; 3; 4; 5; 10; 20; 50; 100; 200; 500; 1,000; 2,000; 5,000; 10,000; 20,000; 50,000; 100,000; 200,000; 300,000; 400,000; 500,000; 600,000; 700,000; 800,000; 900,000; 1,000,000; 2,000,000 or 3,000,000 different SNPs.

In some cases, the methods described herein are used to detect and/or quantify gene expression. In some embodiments, the methods described herein provide high discriminative and quantitative analysis of multiple genes. The methods described herein can discriminate and quantitate the expression of at least 1, 2, 3, 4, 5, 10, 20, 50, 100, 200, 500, 1,000, 2,000, 5,000, 10,000, 20,000, 50,000, 100,000, different target nucleic acids. In some instances, the target nucleic acids are DNA molecules. The DNA molecules may comprise introns and/or exons. The DNA molecules may be cDNA molecules. Alternatively, the target nucleic acids are RNA molecules. In some instances, the RNA molecules are mRNA molecules. The mRNA molecules may be immature mRNA molecules. Alternatively, the mRNA molecules are mature mRNA molecules. The DNA and/or RNA molecules may be from a subject. Alternatively, or additionally, the DNA and/or RNA molecules may be from a foreign source.

Gene expression of one or more DNA and/or RNA molecules can be used to determine the health of particular cells, tissues, or organs. For example, some genes may only be expressed, or may be primarily expressed, in heart tissue, and the quantification of RNA from these genes would give a signal regarding the status of the heart. In some instances, the cell, tissue or organ is from a transplant donor. Alternatively, the cell, tissue, or organ is from the subject. Gene expression of one or more DNA and/or RNA molecules can be used to determine the health of a subject. For example, gene expression of one or more DNA and/or RNA molecules from a cancerous cell can be used to determine the health of a subject suffering from a cancer. In another example, gene expression of one or more DNA and/or RNA molecules from a pathogen and/or pathogen-infected subject can be used to determine the health of a subject suffering from a pathogenic infection. Alternatively, gene expression of one or more DNA and/or RNA molecules can be used to determine the health of a fetus. The signal may comprise the presence/absence of RNAs from a particular gene or several genes. The signal may also represent an increase, or decrease, in the level of a particular gene or several genes.

In some embodiments, the methods described herein are used to detect and/or quantify gene expression of genes with similar sequences. The methods described herein can discriminate and quantitate the expression of genes varying by 1 nt or more than 1, 2, 3, 5, 10, 15, 20, 21, 22, 24, 25, 30, 40, 50, 60, 70, 80, 90, 100, 200, 300, 400 or 500 nt.

In some embodiments, the methods described herein are used to detect and/or quantify genomic DNA regions by mapping the region to the genome of a species in the case where the transplant donor and the transplant recipient are not from the same species (e.g., xenotransplants). In some embodiments, the methods described herein can discriminate and quantitate a DNA region from a species. The methods described herein can discriminate and quantitate of at least 1; 2; 3; 4; 5; 10, 20; 50; 100; 200; 500; 1,000; 2,000; 5,000; 10,000, 20,000; 50,000; 100,000; 200,000; 300,000; 400,000; 500,000; 600,000; 700,000; 800,000; 900,000; 1,000,000; 2,000,000 or 3,000,000 DNA regions from a species.

In some instances, the foreign molecules are detected in a multiplexed reaction. For example, at least about 2, at least about 3, at least about 4, at least about 5, at least about 10, at least about 15, at least about 20, at least about 30, at least about 40, at least about 50, at least about 100, at least about 200, at least about 300, at least about 400, at least about 500, at least about 600, at least about 700, at least about 800, at least about 900, or at least about 1000 molecules are detected in a single reaction or a single reaction container. In another example, at least about 2000, at least about 5000, at least about 10000, at least about 15000, at least about 20000, at least about 30000, at least about 40000, at least about 50000, at least about 100000, at least about 200000, at least about 300000, at least about 400000, at least about 500000, at least about 600000, at least about 700000, at least about 800000, at least about 900000, or at least about 1000000 molecules are detected in a single reaction or a single reaction container.

Detection of the foreign molecules may comprise the detection of genetic variants. In some instances, at least about 2, at least about 3, at least about 4, at least about 5, at least about 10, at least about 15, at least about 20, at least about 30, at least about 40, at least about 50, at least about 100, at least about 200, at least about 300, at least about 400, at least about 500, at least about 600, at least about 700, at least about 800, at least about 900, or at least about 1000 genetic variants are detected in a single reaction. In another example, at least about 2000, at least about 5000, at least about 10000, at least about 15000, at least about 20000, at least about 30000, at least about 40000, at least about 50000, at least about 100000, at least about 200000, at least about 300000, at least about 400000, at least about 500000, at least about 600000, at least about 700000, at least about 800000, at least about 900000, or at least about 1000000 genetic variants are detected in a single reaction.

In some instances, detection of molecules comprises physically separating the molecules into a single target molecule in a reaction. Alternatively, the detection of molecules does not comprise dilution or distribution of the target molecules in the sample into discrete sub-samples or individual molecules. Often, detection of the target molecules occurs in a single reaction volume. The target molecules can be detected simultaneously or sequentially. In some instances, target molecules derived from a foreign genotype are detected. Alternatively, target molecules from multiple genotypes are detected. For example, foreign molecules and subject molecules are detected.

In some instances, the presence or absence of molecules is determined by the use of an integral detector. Generally, an integral detector measures the accumulated quantity of sample component(s) that reach the detector. Alternatively, the presence or absence of molecules is determined by the use of a differential detector. Generally, differential detection is an encoding and detection technique that uses phase changes in the carrier to signal binary ones and zeros.

### VII. Heterogeneous Samples

The present disclosure provides methods and compositions for the detection of foreign molecules within a heterogeneous sample (e.g., a sample comprising at least two different genomic sources). Heterogeneous samples may be from a transplant recipient, a chimeric individual, a subject suffering from cancer, a subject suffering from a disease or condition caused by a pathogen, or a subject with a different disease, disorder or condition.

The heterogeneous sample may be from a tissue, organ, or bodily fluid of a subject. The heterogeneous biological sample can be blood, a blood fraction (e.g., plasma, serum), saliva, sputum, urine, semen, transvaginal fluid, vaginal flow, cerebrospinal fluid, brain fluid, sweat, breast milk, breast fluid (e.g., breast nipple aspirate), stool, bile, secretions, lymph fluid, tears, ear flow, lymph, bone marrow suspension, or ascites. Preferably, the sample is from blood. In some instances, the biological sample is from whole blood, plasma, or serum. In some cases, the biological sample is urine.

In some cases, the heterogeneous biological sample is derived from secretions of the respiratory, intestinal or genitourinary tracts or from a lavage of a tissue or organ (e.g. lung) or tissue which has been removed from organs. The heterogeneous biological sample may be a cell or a tissue biopsy, or a sample taken as a smear. In a particular embodiment, the biological sample is drawn blood and circulating nucleic acids (or other molecules such as proteins) from different genomic sources is found in the blood or plasma, rather than in cells.

The heterogeneous sample can comprise a mixture of molecules (e.g., genetic material, nucleic acids, proteins) from at least two different genomic sources. The different genomic sources contributing the genetic material or other molecules to the biological sample can be from any of the following sets of sources: a pregnant female and a fetus; a non-cancerous cell or a tissue and a cancerous cell or tissue; a donor tissue and a transplant recipient tissue; a healthy cell or tissue and diseased cell or tissue; or from a pathogen (e.g, virus, bacterium, fungus) and an infected subject.

In some cases, the heterogeneous samples are from a chimeric individual. The chimeric individual may be a pregnant subject and the heterogeneous sample may comprise a foreign molecule from a fetus and a molecule from the pregnant subject. In other instances, the chimeric individual is a recipient of a blood transfusion and the heterogeneous sample comprises a foreign molecule from a blood donor and a molecule from the recipient.

The sample can be obtained by a health care provider, for example, a physician, physician assistant, nurse, veterinarian, dermatologist, rheumatologist, dentist, paramedic, or surgeon. The sample can be obtained by a research technician. In some cases, information related to the sample such as the name of the subject, gender, ethnicity, national origin, race, disease-status, site where sample was obtained, name of person who obtained the sample, etc. is entered into a computer or database. In some cases, the information is transmitted to a different location.

In some instances, more than one sample from a subject is obtained. In some instances, at least 2, 3, 4, 5, 6, 7, 8, 9, 10, 11, 12, 13, 14, 15, 20, 25, or 30 samples are obtained from the subject. In some instances, the multiple samples are obtained over a period of time. For example, the multiple samples are obtained over a 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30-week period. Alternatively, the multiple samples are obtained over a 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30-month period. In some instances, the multiple samples are obtained over a 1, 1.5, 2, 2.5, 3, 3.5, 4, 4.5, 5, 6, 7, 8, 9, or 10-year period. In some instances, the multiple samples are obtained about every year, 6-months, 4-months, 3-months, 2-months, 1-month, 4-weeks, 3-weeks, 2-weeks, week, 4-days, 3-days, 2-days, day, 24-hours, 20-hours, 15-hours, 12-hours, 10-hours, 8-hours, 6-hours, 4-hours, 3-hours, 2-hours, or hour.

The biological sample may optionally be enriched for nucleic acid from one or more of the contributing genomic sources using techniques described herein. In the methods of the provided invention, the amount of RNA or DNA from a subject that can be analyzed includes, for example, as low as a single cell in some applications (e.g., a calibration test) and as many as 10 millions of cells or more translating to a range of DNA of 6 pg-60 ug, and RNA of approximately 1 pg-10 ug.

In some embodiments, less than 1 pg, 5 pg, 10 pg, 20 pg, 30 pg, 40 pg, 50 pg, 100 pg, 200 pg, 500 pg, 1 ng, 5 ng, 10 ng, 20 ng, 30 ng, 40 ng, 50 ng, 100 ng, 200 ng, 500 ng, 1 ug, 5 ug, 10 ug, 20 ug, 30 ug, 40 ug, 50 ug, 100 ug, 200 ug, 500 ug or 1 mg of nucleic acids are obtained from the heterogeneous biological sample for genotyping analysis. In some cases, about 1-5 pg, 5-10 pg, 10-100 pg, 100 pg -1 ng, 1-5 ng, 5-10 ng, 10-100 ng, 100 ng-1 ug of nucleic acids are obtained from the sample for genotyping analysis.

### VIII. Exemplary Methods

In some instances, the method comprises generally the steps of: (a) providing a heterogeneous sample from a subject in need thereof; (b) conducting a reaction on the heterogeneous sample to detect one or more foreign molecules; (c) optionally, diagnosing, predicting, or monitoring a status or outcome of a disease or condition based on the detection of one or more foreign molecules; and (d) optionally, determining a therapeutic regimen based on the detection of one or more foreign molecules.

In some instances, the method comprises (a) providing a biological sample containing genetic material from different genomic sources (e.g., a heterogeneous biological sample); (b) optionally, isolating one or more molecules (e.g., DNA, RNA or genomic DNA) from the heterogeneous biological sample; (c) optionally, amplifying the isolated nucleic acid molecule; (d) optionally, directly sequencing the isolated nucleic acid without diluting the genetic material within the sample or distributing the mixture of genetic material into discrete reaction samples; (e) optionally, counting the number of sequences for each genome in the heterogeneous sample; and (f) optionally, conducting an analysis that compares the ratios of the various unique sequences to determine relative amounts of the unique sequences and/or different genomes in the heterogeneous biological sample. Counting the number of sequences for each genome may be achieved via sequence reads. Alternatively, counting the number of sequences for each genome may comprise labeling the molecules. Labeling may comprise the use of barcodes. The method may further comprise mapping one or more unique sequences to one or more genomes represented within the sample. The methods may further comprise diagnosing a disease or condition, predicting the status or outcome of a disease or condition, monitoring the status or outcome of a disease or condition, differentially diagnosing the origin of a graft injury, or determining a therapeutic regimen. In some instances, the methods further comprise the use of a computer, computer software, and/or algorithm for analyzing one or more molecules in the sample. In other instances, the methods further comprise generating a report

The reaction may comprise sequencing the foreign molecules. Alternatively, the reaction comprises hybridizing the foreign molecules to an array. In some instances, the reaction comprises quantifying the foreign molecules. Quantifying the foreign molecules may comprise determining the absolute amount of a foreign molecule. Quantifying the foreign molecules may comprise a comparative method or a non-comparative method. Quantifying the foreign molecules may comprise quantitative PCR. Alternatively, quantifying the foreign molecules may comprise labeling the foreign molecules with barcodes or labels. The reaction may comprise generating a size profile of the foreign molecules. In some instances, the reaction comprises amplifying the foreign molecules. Amplification may comprise a PCR-based method or a non-PCR based method. Alternatively, the reaction may comprise an amplification-free reaction. The reaction may comprise hybridizing the foreign molecules to a solid support. In some instances, the solid support is a microarray. In some instances, the solid support is a bead. The reaction may comprise a multiplex reaction. Alternatively, the reaction may comprise two or more sequential reactions.

In some instances, the disease or condition is organ transplant rejection. The disease or condition may be a cancer. Alternatively, the disease or condition is fetal genetic disorder. The disease or condition may also be pathogenic infection. Examples of pathogenic infections include, but are not limited to, bacterial infections, viral infections, fungal infections, and protozoan infections.

Determining a therapeutic regimen may comprise administering a therapeutic drug. Alternatively, determining a therapeutic regimen comprises modifying, continuing, or initiating a therapeutic regime. Alternatively, determining a therapeutic regimen comprises treating the disease or condition. In some instances, the therapy is an immunosuppressive therapy, anticancer therapy, or antimicrobial therapy. In other instances, diagnosing, predicting, or monitoring a disease or condition comprises determining the efficacy of a therapeutic regimen. Diagnosing, predicting, or monitoring a disease or condition comprises determining drug resistance. In some instances, monitoring a disease or condition comprises detecting transplant rejection. Predicting a disease or condition can comprise predicting the risk of a transplant rejection. Diagnosing a disease or condition may comprise diagnosing a fetal genetic disorder. In some instances, predicting a disease or condition comprises determining the risk of a fetal genetic disorder.

### IX. Performance

In some embodiments, the invention provides highly sensitive, non-invasive diagnostics for monitoring the health of a transplanted organ and managing the overall-health of the recipient using partial or whole genome analysis of circulating nucleic acids derived from tumors as compared to the patient's genome. Often, the methods of the invention deliver a higher detection rate of molecules (e.g., circulating nucleic acids, foreign molecules) in a host subject. For example, the methods of the invention may detect at least about 1.5-fold, 2-fold, 3-fold, 4-fold, 5-fold, 6-fold, 7-fold, or 10-fold more molecules than current methods.

The methods of the invention often provide improved clinical performance compared to conventional screening methods. In some instances, improved clinical performance comprises earlier diagnosis of a disease or condition. Alternatively, improved clinical performance comprises improved prediction of a status or outcome.

In some instances, the accuracy of the diagnosis, prediction, or monitoring a status or outcome of a disease or condition is at least about 50%, at least about 55%, at least about 60%, at least about 65%, at least about 70%, or at least about 75%. Preferably, the accuracy of the diagnosis, prediction, or monitoring of a disease or condition is at least about 80%, at least about 85%, at least about 90%, at least about 95%, or at least about 97%.

In some instances, the methods, compositions, and systems disclosed herein further comprise the use of a proportional-integral-derivative controller (PID controller). Generally, a PID controller is a generic control loop feedback mechanism (controller). A PID controller can calculate an "error" value as the difference between a measured process variable and a desired setpoint. The controller can attempt to minimize the error by adjusting the process control inputs. Generally, the PID controller calculation (algorithm) involves three separate constant parameters, and is accordingly sometimes called three-term control: the proportional, the integral and derivative values, denoted P, I, and D. These values can be interpreted in terms of time: P depends on the present error, I on the accumulation of past errors, and D is a prediction of future errors, based on current rate of change. The weighted sum of these three actions can be used to adjust the process via a control element.

Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. The term "about" as used herein refers to a range that is 15% plus or minus from a stated numerical value within the context of the particular usage. For example, about 10 would include a range from 8.5 to 11.5.

### Examples

### Example 1. Differential diagnosis of the origin of a graft injury

In response to a rise in donor DNA levels, differential diagnosis of the origin of graft injury can be performed by looking at the size profile of donor DNA molecules identified in the cell-free fraction. Cell-free DNA is released from both apoptotic and necrotic cells, but the size distribution of DNAs differs in these two cases. Apoptotic cell death involves nuclease digestion of the genomic DNA while still bound to nucleosomes prior to release from the cell. Consequently, apoptotic contributions to the cell-free DNA are small fragments that form a ladder of sizes starting around 180 bp, then 360 bp, then 540 bp, and so on with the majority of molecules at the smallest sizes. Necrotic cell death is not as orderly and the released DNA is generally of a larger size, and is not digested into a smooth ladder of sizes but instead is a smear. Different causes of graft injury can give a different proportion of apoptotic and necrotic cell death. Infectious pathogens, for example, often express RNA messages that turn off cellular apoptotic processes, and increased necrotic contribution of cellular DNA is expected in such cases.

To assay the size distribution of donor DNA molecules, paired-end sequencing of cell-free DNA libraries made from fluids, including but not limited to plasma and urine, is performed. These libraries may be prepared with size selection, to assay smaller/larger ranges than normal, or without additional size selection after preparation. Following sequencing, the recipient/donor SNP approach is used to identify all molecules of donor origin, and the size of the inserts for this population calculated from the paired-end sequence alignment. A histogram of sizes can be made and features of this distribution, including mean/median size and the extent of apoptotic ladder-like patterning, is used to determine the ratio of apoptotic versus necrotic contribution. This ratio is used (possibly in combination with other signals including overall donor DNA levels, sequences from an infectious agent, or sequences from the immune repertoire) to perform the differential diagnosis of different causes of graft injury.

### Example 2. Predicting transplant rejection

A blood sample from a transplant recipient is analyzed for donor-derived DNA in order to predict a risk of transplant rejection. The donor-derived cell-free DNA is detected via sequencing. Long-sequencing technology is used to sequence at least about 1500 bp of the donor-derived cell-free DNA. The amount of donor-derived cell-free DNA is quantified by counting the number of sequence reads. A transplant rejection is predicted if the total percentage of the donor-derived cell-free DNA is greater than about 1% of the total DNA in the sample.

### Example 3. Modifying an immunosuppressive regimen

A urine sample from a liver transplant recipient treated with an immunosuppressive regimen is analyzed for donor-derived DNA in order to monitor an immunosuppressive regimen. A small fragment sample is generated by isolating DNA fragments less than about 150 base pairs from the urine sample. The amount of donor-derived DNA in the small fragment sample is determined by quantitative PCR. The amount of donor-derived DNA is less than about 0.5% of the total DNA in the small fragment sample, indicating that the donor graft is healthy. As a result of the healthy graft, one or more immunosuppressive drugs in the immunosuppressive regimen are reduced.

Alternatively, multiple urine samples are obtained from a heart transplant recipient treated with an immunosuppressive regimen are analyzed for kidney-derived DNA in order to monitor an immunosuppressive regimen. The urine samples are collected at different time points (e.g., 0 days, 7 days, 14 days, and 21 days after administration of an immunosuppressive regimen). For each urine sample at each time point, a large fragment sample is generated by isolating DNA fragments greater than about 150 base pairs from each urine sample. The amount of kidney-derived DNA in the large fragment sample is determined by digital PCR. The amount of kidney-derived DNA in the large fragment increases over time, indicating increased drug nephrotoxicity. As a result of the nephrotoxicity, one or more immunosuppressive therapies in the immunosuppressive regimen are reduced or removed from the regimen. Optionally, one or more immunosuppressive therapies in the immunosuppressive regimen are replaced with an alternative therapy.

### Example 4. Determining an anti-cancer regimen

A serum sample from a subject suffering from a breast cancer is analyzed for cancer cell-derived RNA. The nucleic acids in the sample are mixed with a plurality of unique barcodes to produce barcoded-RNA molecules. The barcoded-RNA molecules are reverse transcribed to produce barcoded-DNA molecules. The barcoded-DNA molecules are sequenced and the quantity of the nucleic acids is determined by counting the number of unique barcodes for each sequence. If the amount of the cancer cell-derived RNA increases by at least about 2-fold, then an anti-cancer regimen is administered or increased.

### Example 5. Identification of drug responders

A urine sample from a subject suffering from a bacterial infection is analyzed for the presence of bacterial DNA. Multiple urine samples are collected from the subject suffering from the bacterial infection. The subject is concurrently treated with an antibiotic and the amount of bacterial DNA in the urine sample is detected over time. If the amount of bacterial DNA in the sample decreases over time, then the subject is identified as a responder to the antibiotic therapy.

### Example 6. Determining an antiviral regimen

A serum sample from a subject suffering from a viral infection is analyzed for the presence of viral RNA. The viral RNA is isolated from the serum sample and reverse transcribed into DNA. The viral DNA is sequenced and the strain of the virus is determined. An antiviral regimen is administered based on the strain of the virus. Seven days after administration of the antiviral regimen, a second serum sample is obtained from the subject. A size profile of the subject-derived DNA is generated. The size profile indicates high necrotic cell death. The antiviral regimen is terminated and a new antiviral regimen is administered. Seven days after administration of the second antiviral regimen, a third serum sample is obtained from the subject. A size profile of the subject-derived DNA is generated. The size profile reveals normal levels of necrotic cell death. The second antiviral regimen is maintained until the viral infection is no longer detected.

## Claims

1. A method comprising the steps of:
a. detecting a quantity of nucleic acids from cancer tissue in a biological sample obtained from a subject with cancer, the biological sample comprising circulating cell-free nucleic acids, wherein the circulating cell-free nucleic acids comprise nucleic acids from cancer tissue and normal tissue, wherein the circulating cell-free nucleic acids are mRNA or DNA; and wherein the subject has been administered a therapeutic regimen, wherein the detecting comprises conducting a sequencing reaction on the circulating cell-free nucleic acids; and
b. modifying the therapeutic regimen to be administered to the subject based on the quantity of the nucleic acids from the cancer tissue in the biological sample, wherein the therapeutic regimen is increased if the quantity of the nucleic acids from the cancer tissue in the biological sample is greater than 0.5% of the total nucleic acids in the biological sample.

2. The method according to claim 1, wherein the percentage of nucleic acids from cancer tissue is determined.

3. A method comprising the steps of:
a. detecting a first quantity of cell-free nucleic acids in a biological sample obtained from a subject with cancer at a first point in time, the biological sample comprising circulating cell-free nucleic acids, wherein the subject has been administered a therapeutic regimen and wherein the circulating cell-free nucleic acids are mRNA or DNA from normal tissue and cancer tissue, wherein the detecting comprises conducting a sequencing reaction on the circulating cell-free nucleic acids;
b. detecting a second quantity of the cell-free nucleic acids from the cancer tissue in a second biological sample obtained from the subject at a second point in time, wherein the second point of time is within a six-month period after the first biological samples are obtained from the subject, wherein the detecting comprises conducting a sequencing reaction on the circulating cell-free nucleic acids; and
c. adjusting the therapeutic regimen to be administered to the subject based on the first and second quantities, wherein the therapeutic regimen is increased if the second quantity of nucleic acids is 2.5-fold greater than the first quantity of nucleic acids.

4. The method of claims 1 to 3, wherein the sequencing reaction is a next generation sequencing reaction.

5. The method of any one of claims 1 to 3, wherein the detecting comprises using a microarray.

6. The method of claim 5, wherein the microarray is a single nucleotide polymorphism array.

7. The method of any one of claims 1 to 3, wherein the circulating nucleic acids comprise DNA.

8. The method of any one of claims 1 to 3, wherein the detecting comprises detecting one or more of the following: a variable number tandem repeat (VNTR), a short tandem repeat (STR), a single nucleotide polymorphism (SNP) pattern, a hypervariable region, a dinucleotide repeat, a trinucleotide repeat, a tetranucleotide repeat, or a simple sequence repeat.

9. The method of any one of claims 1 to 3, wherein the therapeutic regimen is a chemotherapeutic regimen, a radiation therapy regimen, a monoclonal antibody regimen, an anti angiogenic regimen, an oligonucleotide therapeutic regimen, or any combination thereof.

10. The method of any one of claims 1 to 3, wherein the detecting comprises detecting a mutation in an oncogene, a microsatellite alteration, or a viral genomic sequence.

11. The method of any one of claims 1 to 3, wherein the sample is a blood sample.

12. The method of any one of claims 1 to 3, wherein the detecting discriminates and quantitates the expression of at least 20 different target nucleic acids.

13. The method of claims 1 to 3, wherein the detecting discriminates a DNA region containing a polymorphism.

14. The method of claims 1 to 3, further comprising inserting the biological sample into a device that generates a size profile of a set of molecules derived from the biological sample.

## Patentansprüche

1. Verfahren, das die folgenden Schritte beinhaltet:
a. Detektieren einer Menge an Nukleinsäuren von einem Krebsgewebe in einer biologischen Probe, die von einem Subjekt mit Krebs erhalten wurde, wobei die biologische Probe zirkulierende, zellfreie Nukleinsäuren beinhaltet, wobei die zirkulierenden, zellfreien Nukleinsäuren Nukleinsäuren von Krebsgewebe und normalem Gewebe beinhalten, wobei die zirkulierenden, zellfreien Nukleinsäuren mRNA oder DNA sind; und wobei dem Subjekt ein therapeutisches Regime verabreicht wurde, wobei das Detektieren das Durchführen einer Sequenzierreaktion an den zirkulierenden, zellfreien Nukleinsäuren beinhaltet; und
b. Modifizieren des an das Subjekt zu verabreichenden therapeutischen Regimes basierend auf der Menge der Nukleinsäuren von dem Krebsgewebe in der biologischen Probe, wobei das therapeutische Regime erhöht wird, falls die Menge der Nukleinsäuren von dem Krebsgewebe in der biologischen Probe größer als 0,5 % der gesamten Nukleinsäuren in der biologischen Probe ist.

2. Verfahren gemäß Anspruch 1, wobei der Prozentwert der Nukleinsäuren vom Krebsgewebe bestimmt wird.

3. Verfahren, das die folgenden Schritte beinhaltet:
a. Detektieren einer ersten Menge an zellfreien Nukleinsäuren in einer biologischen Probe, die von einem Subjekt mit Krebs zu einem ersten Zeitpunkt erhalten wurde, wobei die biologische Probe zirkulierende, zellfreie Nukleinsäuren beinhaltet, wobei dem Subjekt ein therapeutisches Regime verabreicht wurde und wobei die zirkulierenden, zellfreien Nukleinsäuren mRNA oder DNA von normalem Gewebe und Krebsgewebe sind, wobei das Detektieren das Durchführen einer Sequenzierreaktion an den zirkulierenden, zellfreien Nukleinsäuren beinhaltet;
b. Detektieren einer zweiten Menge der zellfreien Nukleinsäuren von dem Krebsgewebe in einer zweiten biologischen Probe, die von dem Subjekt zu einem zweiten Zeitpunkt erhalten wurde, wobei der zweite Zeitpunkt innerhalb einer Sechs-Monate-Periode nach Erhalten der ersten biologischen Proben von dem Subjekt liegt, wobei das Detektieren das Durchführen einer Sequenzierreaktion an den zirkulierenden, zellfreien Nukleinsäuren beinhaltet; und
c. Anpassen des an das Subjekt zu verabreichenden therapeutischen Regimes basierend auf der ersten und zweiten Menge, wobei das therapeutische Regime erhöht wird, falls die zweite Menge an Nukleinsäuren 2,5-fach größer als die erste Menge an Nukleinsäuren ist.

4. Verfahren gemäß Ansprüchen 1 bis 3, wobei die Sequenzierreaktion eine Sequenzierreaktion der nächsten Generation ist.

5. Verfahren gemäß einem der Ansprüche 1 bis 3, wobei das Detektieren das Verwenden eines Mikroarrays beinhaltet.

6. Verfahren gemäß Anspruch 5, wobei das Mikroarray ein Einzelnukleotid-Polymorphismus-Array ist.

7. Verfahren gemäß einem der Ansprüche 1 bis 3, wobei die zirkulierenden Nukleinsäuren DNA beinhalten.

8. Verfahren gemäß einem der Ansprüche 1 bis 3, wobei das Detektieren das Detektieren von einem oder mehreren der Folgenden beinhaltet: einer Tandemwiederholung mit variabler Anzahl (VNTR, variable number tandem repeat), einer kurzen Tandemwiederholung (STR, short tandem repeat), einem Einzelnukleotid-Polymorphismus(SNP)-Muster, einer hypervariablen Region, einer Dinukleotidwiederholung, einer Trinukleotidwiederholung, einer Tetranukleotidwiederholung oder einer einfachen Sequenzwiederholung.

9. Verfahren gemäß einem der Ansprüche 1 bis 3, wobei das therapeutische Regime ein chemotherapeutisches Regime, ein Strahlungstherapieregime, ein monoklonales Antikörperregime, ein anti-angiogenes Regime, ein oligonukleotidtherapeutisches Regime oder eine Kombination davon ist.

10. Verfahren gemäß einem der Ansprüche 1 bis 3, wobei das Detektieren das Detektieren einer Mutation in einem Onkogen, einer Mikrosatellitenänderung oder einer viralen genomischen Sequenz beinhaltet.

11. Verfahren gemäß einem der Ansprüche 1 bis 3, wobei die Probe eine Blutprobe ist.

12. Verfahren gemäß einem der Ansprüche 1 bis 3, wobei das Detektieren die Expression von mindestens 20 verschiedenen Zielnukleinsäuren unterscheidet und quantifiziert.

13. Verfahren gemäß Ansprüchen 1 bis 3, wobei das Detektieren eine DNA-Region, die einen Polymorphismus enthält, unterscheidet.

14. Verfahren gemäß Ansprüchen 1 bis 3, das ferner das Einführen der biologischen Probe in eine Vorrichtung beinhaltet, die ein Größenprofil eines Satzes Moleküle erzeugt, die von der biologischen Probe abgeleitet sind.

## Revendications

1. Procédé comprenant les étapes :
a. de détection d'une quantité d'acides nucléiques de tissu cancéreux dans un échantillon biologique obtenu à partir d'un sujet atteint de cancer, l'échantillon biologique comprenant des acides nucléiques exempts de cellules en circulation, dans lequel les acides nucléiques exempts de cellules en circulation comprennent des acides nucléiques de tissu cancéreux et de tissu normal, dans lequel les acides nucléiques exempts de cellules en circulation sont de l'ARNm ou de l'ADN ; et dans lequel un régime thérapeutique a été administré au sujet, dans lequel la détection comprend la direction d'une réaction de séquençage sur les acides nucléiques exempts de cellules en circulation ; et
b. de modification du régime thérapeutique devant être administré au sujet sur la base de la quantité des acides nucléiques du tissu cancéreux dans l'échantillon biologique, dans lequel le régime thérapeutique est augmenté si la quantité d'acides nucléiques du tissu cancéreux dans l'échantillon biologique est supérieure à 0,5 % du total des acides nucléiques dans l'échantillon biologique.

2. Procédé selon la revendication 1, dans lequel le pourcentage d'acides nucléiques de tissu cancéreux est déterminé.

3. Procédé comprenant les étapes :
a. de détection d'une première quantité d'acides nucléiques exempts de cellules dans un échantillon obtenu à partir d'un sujet atteint de cancer à un premier point dans le temps, l'échantillon biologique comprenant des acides nucléiques exempts de cellules en circulation, dans lequel un régime thérapeutique a été administré au sujet et dans lequel les acides nucléiques exempts de cellules en circulation sont de l'ARNm ou de l'ADN de tissu normal et de tissu cancéreux, dans lequel la détection comprend la direction d'une réaction de séquençage sur les acides nucléiques exempts de cellules en circulation ;
b. de détection d'une deuxième quantité d'acides nucléiques exempts de cellules du tissu cancéreux dans un deuxième échantillon biologique obtenu à partir du sujet à un deuxième point dans le temps, dans lequel le deuxième point dans le temps est dans les limites d'une période de six mois après que les premiers échantillons biologiques ont été obtenus à partir du sujet, dans lequel la détection comprend la direction d'une réaction de séquençage sur les acides nucléiques exempts de cellules en circulation ; et
c. d'ajustement du régime thérapeutique devant être administré au sujet sur la base des première et deuxième quantités, dans lequel le régime thérapeutique est augmenté si la deuxième quantité d'acides nucléiques est 2,5 fois plus grande que la première quantité d'acides nucléiques.

4. Procédé selon les revendications 1 à 3, dans lequel la réaction de séquençage est une réaction de séquençage de nouvelle génération.

5. Procédé selon l'une quelconque des revendications 1 à 3, dans lequel la détection comprend l'utilisation d'une biopuce.

6. Procédé selon la revendication 5, dans lequel la biopuce est une biopuce à polymorphisme nucléotidique unique.

7. Procédé selon l'une quelconque des revendications 1 à 3, dans lequel les acides nucléiques en circulation comprennent de l'ADN.

8. Procédé selon l'une quelconque des revendications 1 à 3, dans lequel la détection comprend la détection d'un ou de plusieurs éléments parmi les suivants : un nombre variable de répétitions en tandem (VNTR), une répétition en tandem courte (STR), un motif de polymorphisme nucléotidique unique (SNP), une région hypervariable, une répétition de dinucléotides, une répétition de trinucléotides, une répétition de tétranucléotides, ou une répétition de séquence unique.

9. Procédé selon l'une quelconque des revendications 1 à 3, dans lequel le régime thérapeutique est un régime de chimiothérapie, un régime de radiothérapie, un régime d'anticorps monoclonal, un régime anti-angiogénique, un régime de thérapie par les oligonucléotides, ou toute combinaison de ceux-ci.

10. Procédé selon l'une quelconque des revendications 1 à 3, dans lequel la détection comprend la détection d'une mutation dans un oncogène, d'une altération de microsatellite, ou d'une séquence génomique virale.

11. Procédé selon l'une quelconque des revendications 1 à 3, dans lequel l'échantillon est un échantillon sanguin.

12. Procédé selon l'une quelconque des revendications 1 à 3, dans lequel la détection exerce une discrimination à l'égard d'au moins 20 acides nucléiques cibles différents et quantifie l'expression d'au moins 20 acides nucléiques cibles différents.

13. Procédé selon les revendications 1 à 3, dans lequel la détection exerce une discrimination à l'égard d'une région d'ADN contenant un polymorphisme.

14. Procédé selon les revendications 1 à 3, comprenant en outre l'insertion de l'échantillon biologique dans un dispositif qui génère un profil de taille d'un ensemble de molécules dérivées de l'échantillon biologique.
